# EUROPEAN PATENT SPECIFICATION

(11) **EP 3 209 680 B1**
(45) Date of publication and mention of the grant of the patent: **17.04.2019**
(21) Application number: 15804943.7
(22) Date of filing: 19.10.2015
(51) Int. Cl.: C07K 14/435, C12N 5/074

(54) **NEW METHODS AND AGENTS FOR CELLULAR REPROGRAMMING**
NEUE VERFAHREN UND MITTEL ZUR ZELLUMPROGRAMMIERUNG
NOUVEAUX PROCÉDÉS ET AGENTS POUR LA REPROGRAMMATION DE CELLULES

(30) Priority: 21.10.2014 IT RM20140596
(43) Date of publication of application: 30.08.2017
(73) Proprietor: Consiglio Nazionale delle Ricerche, 00185 Roma (IT)
(72) Inventor: ZUCCHI, Ileana, I-20090 Segrate (MI) (IT); TRIA, Valeria, I-20090 Segrate (MI) (IT); REINBOLD, Rolland Alvons, I-20090 Segrate (MI) (IT)
(74) Representative: Predazzi, Valentina
(86) International application number: PCT/IB2015/058022
(87) International publication number: WO 2016/063192

(56) References cited:
- WO-A2-2012/061075
- KAZUTOSHI TAKAHASHI ET AL: "Induction of Pluripotent Stem Cells from Adult Human Fibroblasts by Defined Factors", CELL, CELL PRESS, US, vol. 131, no. 5, 30 November 2007 (2007-11-30), pages 861-872, XP008155962, ISSN: 0092-8674, DOI: 10.1016/J.CELL.2007.11.019 cited in the application
- KAZUTOSHI TAKAHASHI ET AL: "Induction of Pluripotent Stem Cells from Mouse Embryonic and Adult Fibroblast Cultures by Defined Factors", CELL, CELL PRESS, US, vol. 126, no. 4, 25 August 2006 (2006-08-25), pages 663-676, XP008157317, ISSN: 0092-8674, DOI: 10.1016/J.CELL.2006.07.024 cited in the application
- JING YANG ET AL: "TRANSLATIONAL AND CLINICAL RESEARCH Tumor Tropism of Intravenously Injected Human-Induced Pluripotent Stem Cell-Derived Neural Stem Cells and Their Gene Therapy Application in a Metastatic Breast Cancer Model Key Words. Pluripotent stem cells @BULLET Neural stem cell @BULLET In vivo tracking @BULLET", STEM CELLS, vol. 30, 6 February 2012 (2012-02-06), pages 1021-1029, XP055189558,
- MAYEUR G L ET AL: "Malignant transformation by the eukaryotic translation initiation factor 3 subunit p48 (eIF3e)", FEBS LETTERS, ELSEVIER, AMSTERDAM, NL, vol. 514, no. 1, 6 March 2002 (2002-03-06) , pages 49-54, XP004597948, ISSN: 0014-5793, DOI: 10.1016/S0014-5793(02)02307-4
- MACK DAVID L ET AL: "Expression of truncated Int6/eIF3e in mammary alveolar epithelium leads to persistent hyperplasia and tumorigenesis", BREAST CANCER RESEARCH, CURRENT SCIENCE, LONDON, GB, vol. 9, no. 4, 12 July 2007 (2007-07-12), page R42, XP021031131, ISSN: 1465-5411, DOI: 10.1186/BCR1742
- DATABASE UniProt [Online] 22 February 2012 (2012-02-22), "SubName: Full=Eukaryotic translation initiation factor 3 subunit E {ECO:0000313|Ensembl:ENSP00000430152}; Flags: Fragment;", XP002754173, retrieved from EBI accession no. UNIPROT:H0YBR5 Database accession no. H0YBR5 -& NUSBAUM C ET AL: "DNA sequence and analysis of human chromosome 8", NATURE, NATURE PUBLISHING GROUP, UNITED KINGDOM, vol. 439, no. 7074, 19 January 2006 (2006-01-19), pages 331-335, XP002365497, ISSN: 0028-0836, DOI: 10.1038/NATURE04406

## Description

The present description relates to a new transcript referred to here as eIF3e-5a, which is an isoform of the human transcript, known in the literature, eukaryotic translation initiation factor 3 sub-unit E full length (EIF3E-FL code UNIPROT P60228- EIF3E_HUMAN last updated on 16 January 2004) encoded by the gene EIF3E alias INT6 (gene ID 3646, mRNA NM_001568.2), the use thereof in cellular reprogramming methods for the production of induced stem cells, the cells obtained from such methods, and use thereof.

### PRIOR ART

The normal homeostasis of an organ or of a tissue and the function thereof are dependent on the presence of tissue-specific stem cells. Regenerative medicine and cellular substitution therapy are based on the reprogramming of cells already differentiated in order to generate *de novo* pluripotent stem cells or somatic stem cells. The stem cells generated using this method are called induced pluripotent cells (iPSCs). Cellular reprogramming involves the generation *in vitro* of iPSC cells or of somatic stem cells from fibroblasts or from other differentiated cells isolated from patients and the reconversion of the iPSCs or of the somatic stem cells into new differentiated cells. Cellular reprogramming may involve both the loss and gain of a diseased state insofar as iPSC cells can also be generated from diseased cells for the purpose of obtaining cellular models of diseases for research purposes or for the experimentation of patient-specific drugs. For example, iPSC cells may be generated from diseased primary cells of patients suffering from Alzheimer's or Parkinson's or from tumour cells or from cancer stem cells.

The development of technologies for therapies based on the substitution of diseased cells and in general for regenerative medicine is still at an early stage insofar as these are dependent on the identification of mechanisms able to effectively induce changes in the state of chromatin and in gene expression and the reversal of the negative effects associated with the ageing in the cells of elderly patients. Recent studies have demonstrated that a prerequisite for cellular reprogramming is the induction of cell proliferation and the conversion of chromatin from a closed chromatin state (heterochromatin) to an open chromatin state (euchromatin). It is also necessary to consider that the cells after a certain number of divisions enter into a phase of senescence, slightly shortening each of the telomeres with each mitosis. Hayflick has in fact demonstrated that a population of normal human foetal cells cultivated *in vitro* can divide between 40 and 60 times (Hayflick L, Moorhead PS. 1961. The serial cultivation of human diploid cell strains. Exp Cell Res 25: 585-621). After such a number of divisions, also referred to as the "Hayflick limit" of proliferation, the shortening of the telomeres makes impossible the cellular division and reprogramming of the state of the chromatin and explains why it is extremely difficult to generate iPSCs from cells isolated from elderly individuals.

The ageing of the cell, which seems to correlate with the general physical ageing of the human body, is a natural mechanism of which the objective is to prevent the development of tumours in aged cells or with damaged DNA. In cases in which the cells avoid senescence and continue to proliferate after the Hayflick limit, they become immortal and acquire the potential to become tumour cells, and, acquiring the capability to proliferate indefinitely, can easily accumulate lesions harmful to the DNA. Embryonic cells and embryonic stem cells, however, are not immortalised cells and also enter into senescence similarly to somatic cells in order to prevent themselves from being able to acquire damage to the DNA and generate tumours due to continuous cell proliferation. An example is constituted by F9 cells, which are immortalised pluripotent cells that form teratocarcinomas when injected into mice, contrary to mouse pluripotent embryonic stem cells (mESCs), which instead form teratomas.

The difficulty in generating iPSC cells from elderly patients lies in the fact that the cells must recover the ability to proliferate, but must also maintain the ability to repair their DNA and to differentiate themselves into fully functional tissue cells. For the *in vitro* formation of iPSC cells, current research shows that telomeres and the state of the chromatin of fibroblasts or of differentiated cells must be reprogrammed to a state similar to that of embryonic stem cells.

Yamanaka described, in 2006, initial methods (Takahashi K. and Yamanaka S. Induction of Pluripotent Stem Cells from Mouse Embryonic and Adult Fibroblast Cultures by Defined Factors Cell 2006, 07:024), and Takahashi K. Tanabe K, Ohnuki M, Narita M, Ichisaka T, Tomoda K, Yamanaka S. Induction of Pluripotent Stem Cells from Adult Human Fibroblasts by Defined Factors Cell 2007, 11: 19) for the generation of IPSC cells used retroviral or lentiviral vectors for the genomic integration of 4 transgenes: Oct3/4, Sox2, Klf4, and c-Myc. The use of viral constructs in medicine has been the topic of discussion since the random integration of the 4 transgenes may lead, in the long term, to the activation or to the inactivation of oncogenes and onco-suppressors, to the inactivation of the promoters thereof, or to the interruption of the encoding regions.

Unfortunately, one of the four genetic sequences (c-Myc) necessary for the induction of pluripotency is cancerogenic, and 20 % of rats with iPS implants develop cancerous teratomas. In a subsequent study, Yamanaka reported that it is also possible to create IPSs without c-Myc. The process requires much time and is not as efficient as the other method, however the resultant chimers do not develop cancer.

Thomson and colleagues (Yu et al, "Induced pluripotent stem cell lines derived from human somatic cells" Science. 2007 Dec 21;318(5858):1917-20. Epub 2007 Nov 20) used OCT4, SOX2, NANOG and a different gene, LIN28, which is believed to be involved in the self-renewal of stem cells, with lentiviral system.

In accordance with that reported by Thomson in European patent application EP2476750 [0115], the lentiviral system applied to the genes used by Yamanata would not have given cellular reprogramming results.

WO2012/061075 discloses the generation of iPSCs from human hematopoietic stem cells using OCT4, SOX2, KLF4, and c-MYC by transduction with an anti-HIV lentiviral vector.

In any case, the use of viral vectors that may lead to the integration of a viral transgene in the genome of human cells is not legally or ethically acceptable for clinical applications, whereas modifications of the epigenomic type, based on transient changes of the state of the chromatin, are currently considered ideal for applications in the medical field.

Recent approaches, based on the transient up-regulation of mRNA of c-Myc, Sox2, Klf4 and Oct3/4 (obtained by transfection), are considered safer for the generation of iPSC cells for clinical applications. In addition, since c-Myc is a well-established oncogene, research is heavily oriented towards the identification of new compounds, small molecules, genes or micro-RNA that could functionally substitute c-Myc in the generation of iPSC cells. In addition, because c-Myc promotes local and global chromosomal changes that have not yet been clearly characterised, it makes the iPSC cells problematic for use in patients due to the possible induction of malignant transformation.

Finally, Mayeurg and Hershey, "Malignant transformation by the eukaryotic translation initiation factor 3 subunit p48 (eIF3e), FEBS Lett. 2002 Mar 6:514(1):49-54 discloses that the e-subunit (p48) of mammalian initiation factor 3 is encoded by the Int6 gene, a common site for integration of the mouse mammary tumor virus genome, leading to the production of a truncated eukaryotic initiation factor-3e (eIF3e). The paper also discloses that stable expression of a truncated eIF3e in NIH 3T3 cells causes malignant transformation and inhibits the onset of apoptosis caused by serum starvation whereas stable expression of full-length eIF3e does not cause transformation.

### SUMMARY OF THE INVENTION

The present invention provides a new transcript not yet described in the literature and referred to here as EIF3E-5a, which is an isoform of the human transcript, known in the literature, EIF3E full length (EIF3E-FL) encoded by the gene EIF3E and formed by exons 1-13 of said gene (gene ID 3646, mRNA NM_001568.2), said isoform being encoded by the nucleotide sequence SEQ ID NO 1 (defined here also as EIF3E-5a) or by other sequences deducible from the amino acid sequence SEQ ID NO 2 and having the amino acid sequence SEQ ID NO 2. The authors of the present invention have discovered that the above-mentioned isoform 5a or nucleic acids encoding same can be used successfully, in combination with other genes, in the cellular reprogramming of somatic cells of humans and of other mammals into IPSCs.

The cDNA sequence encoding the isoform of the invention (SEQ ID NO 1) and the putative sequence of the protein derived therefrom (SEQ ID NO 2) were filed in confidential form at GenBank on 13 December 2012 and will be made available to the public only after 31 December 2014.

The authors of the present invention, after having studied the properties of the above-mentioned transcript, discovered that the use of the human transcript EIF3E-5a (SEQ ID NO 2) induces a rise in cell proliferation and the remodelling of the chromatin into fibroblasts or other types of cells isolated from human tissues or tissues from other mammals and makes it possible to promote the generation, on the basis of mammalian somatic cells, of induced pluripotent stem cells (IPSCs).

The cellular reprogramming of the isoform of the present invention has been performed both with the conventional approach described in the literature, which involves the use of lentivirus, and with transient transfection methods with mRNA by way of substitution of the technology with lentivirus with the objective of avoiding problems of low safety linked with the use of viral constructs. It is also possible to execute the protocol by inserting the nucleic acids of interest into non-viral episomal vectors, although the use of such vectors has normally proven to be less effective. In the methods of the present invention nucleotides for the down-regulation of particular sequences have also been used optionally.

The data obtained by the authors of the present invention supports the fact that the transient expression of EIF3E-5a does not convert healthy cells into tumour cells and does not promote the induction of tumours when non-tumour cells transduced with EIF3E-5a transplanted into nude mice have not generated tumours. EIF3E-5a does not act here as an oncogene. Instead, it has been observed that the rise in the cell proliferation is only temporary when the expression of EIF3E-5a is transient and that the transient remodelling of the chromatin obtained with the method provided by the inventors did not produce, in the long-term culture, a rise of the rate of mutation of the normal primary cells.

The present invention therefore describes the use of nucleotide molecules EIF3E-5a, having SEQ ID NO 1, or of molecules with nucleotide sequences encoding the protein EIF3E-5a (SEQ ID NO 2), or of the protein EIF3E-5a itself (SEQ ID NO 2) in regenerative medicine or in therapies based on cellular substitution insofar as these are safer than the proto-oncogene c-MYC.

It is important to highlight that, since the existing technologies for the programming of cells are more effective if cells of young patients are used, there is a great need for new and effective technologies for the generation of iPSC cells for elderly patients.

The inventors have observed that the down-regulation of EIF3E-FL makes it possible to cancel the state of cellular senescence and have thus provided a cellular reprogramming method applicable in particular to senescent cells or to cells of elderly patients, in which the up-regulation of EIF3E-5a and the down-regulation of EIF3E-FL are combined and the efficacy of the reprogramming, even of cells isolated from elderly patients, is maximised.

It is noted that the gene EIF3E (gene ID 3646, mRNA NM_001568.2) and its transcript EIF3E in its complete form, (also indicated here as INTL6-FL) are described in US006255105B1 and US7067635B2. Both documents describe the isolation of the human and murine gene encoding EIF3E in full form, or rather with all the exons (also defined in the present description as EIF3E-FL), and the use of reagents derived from nucleotide and amino acid sequences of the gene itself for diagnostic methods, immunotherapy, gene therapy for substitution of ineffective forms of the protein EIF3E and vaccines, but do not describe the truncated form of the gene (SEQ ID NO 1) and the protein deriving therefrom, described here as EIF3E-5a (SEQ ID NO 2), nor do they describe or suggest the use of reagents derived from INT6 FL or EIF3E-FL (the terms INT6 and EIF3E are interchangeable throughout the description in respect of the gene) generally as tools for promoting cellular reprogramming and remodelling of chromatin or for inducing the generation of iPSC cells or of somatic stem cells for regenerative medicine and therapy based on cellular substitution, or for the generation of cellular disease models.

The present invention therefore relates to a molecule of nucleic acids having SEQ ID NO 1 or 19 encoding an isoform that does not comprise exons 6-13 of the human gene EIF3E, a protein having SEQ ID NO 2 in which said protein is a short isoform that does not include the transcript of exons 6-13 and instead includes part of the intron 5 of the human gene EIF3E, an agent for the production of IPSC cells from human or non-human mammalian somatic cells including the protein having SEQ ID NO 2, or rather a molecule of nucleic acid having SEQ ID NO 1 or 19 or a nucleic acid encoding the protein having SEQ ID NO 2; an *in vitro* method for the production of induced pluripotent stem cells (IPSC) from somatic cells comprising the following steps:
a. introducing a protein as represented in SEQ ID NO 2 or a nucleic acid molecule encoding it and, concomitantly or subsequently, the human transcription factor SOX-2, the human Krueppel-like factor 4, the POU domain, class 5. transcription factor 1 and LIN28 or nucleic acids encoding them, in a human or in a non-human mammalian somatic cell, and
d. culturing said cell until it divides into a population of pluripotent cells optionally further comprising step b. before, concomitantly or subsequently to a. and before d
b. introducing the Myc proto-oncogene protein or a nucleic acid encoding it in said human or non-human mammalian somatic cell in a passage of cellular reprogramming and/or optionally further comprising step c. before, concomitantly or subsequently to a. and optionally c. before d .:
c. introducing a down-regulator specific for EIF3E Full length which does not interfere with the regulation of the nucleic acid molecule as represented in SEQ ID NO 1 or 19, and/or of the protein as represented in SEQ ID NO 2 in a passage of cellular reprogramming, wherein said EIF3E Full length down-regutator is a nucleic acid selected from a siRNA, a shRNA a miRNA, an antibody specific for EIFE3E FL or fragments thereof that do not react with EIF3E-5a; to an *in vitro* method for the production of differentiated somatic cells comprising the steps of
   a. producing human or non-human mammalian induced pluripotent stem cells by way of the method according to any one of claims 11 to 16 and
   b. submitting the induced pluripotent stem cells thus obtained to a protocol of cell differentiation;
   to the use of the protein having SEQ ID NO 2 or of the nucleic acid molecule having SEQ ID NO 1 or 19, or of other nucleic acid molecules of which the sequences encode for said protein for the generation of human or non-human mammalian induced pluripotent stem cells (IPSC) obtainable by the methods according to the invention for use in therapies that require cellular reprogramming; to an *in vitro* method for the production of induced pluripotent stem cells (IPSC) from senescent somatic cells or from somatic cells taken from elderly individuals comprising the use of a down-regulator specific for EIF3E Full Length which does not interfere with the regulation of the molecules having SEQ ID NO 1 or 19 and/or SEQ ID NO 2 and of a reagent for the up-regulation of the EIF3E-5a messenger.

### GLOSSARY

Induced pluripotent stem cells (iPSCs).

Mouse embryonic stem cells (mESCs)

Euchromatin, in accordance with the definition commonly used in genetic and cytogenetic texts, in brief: conformation of the less condensed chromatin, typical of cells in active transcription.

Heterochromatin in accordance with the definition commonly used in genetic and cytogenetic tests, in brief: densely packed chromosomal material typical of chromosomal regions not actively transcribed.

Cellular reprogramming: methods that make it possible to return cells already differentiated to undifferentiated or partially differentiated stages.

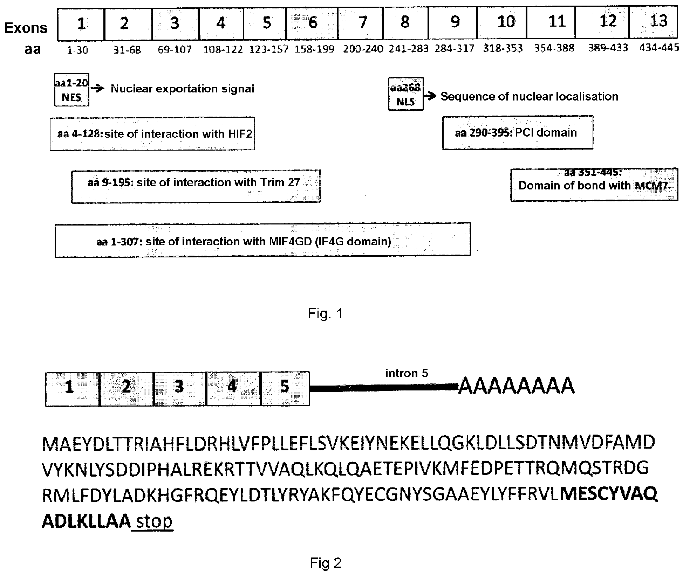

EIF3E Full Length:
mRNA and protein encoded by EIF3E (gene ID 3646, mRNA NM_001568.2) exons 1-13, shown schematically in Figure 1.

EIF3E 5a:
isoform deleted from exon 5 of EIF3E full length, having nucleotide sequence SEQ ID NO 1 or 19 and having amino acid sequence SEQ ID NO 2, shown schematically in Figure 2.

Human transcription factor SOX-2:
P48431- SOX2_HUMAN Protein name: Transcription factor SOX-2; Gene name: SOX2; Gene ID: 6657, updated 5 October 2014; Official gene symbol: SOX2; official full gene name: SRY (sex determining region Y)-box 2; RefSeq of the RNA: NM_003106.3, 2520 bp mRNA linear PRI updated 5 May 2014; RefSeq_of the protein: NP_003097.1, 317 aa linear, PRI last updated 5 May 2014.

Human Krueppel-like factor 4:
O43474- KLF4_HUMAN Protein name: Krueppel-like factor 4; Gene names: KLF4, EZF, GKLF; Gene ID: 9314, last updated 5 October 2014; Official gene symbol: KLF4; official full gene name: Krueppel-like factor 4 (good); RefSeq_of the RNA: NM_004235.4 2949 bp mRNA linear PRI last updated 25 May 2014; RefSeq_of the protein: NP_004226.3 479 aa linear PRI last updated 25 May 2014

POU domain, class 5, transcription factor 1:
Q01860- PO5F1_HUMAN Protein name: POU domain, class 5, transcription factor 1; Gene names: POU5F1, OCT3, OCT4, OTF3; Gene ID: 5460, last updated 5 October 2014; Official symbol of the gene: POU5F1; Official name of the gene: POU class 5 homeobox 1; RefSeq_of the RNA: NM_001173531.2, 1589 bp mRNA linear PRI last updated 4 May 2014; RefSeq_of the protein: NP_001167002 190 aa, linear PRI last updated 4 May 2014.

LIN28:
LIN28 Gene ID: 79727; last updated 8 October 2014; Official symbol: LIN28A; official full gene name: lin-28 homolog A (C. elegans); RefSeq_of the RNA: NM_024674.4, 4014 bp mRNA linear PRI last updated 25 May 2014; RefSeq_of the protein: NP_078950.1, 209 aa linear PRI last updated 25 May 2014

For the purposes of the present description, an agent for the production of IPSC cells from human or non-human mammalian somatic cells means a component or a group of components of which the use, in known cellular reprogramming cultures, contributes to the restoration of the pluripotent state of somatic cells already differentiated, said agent for example potentially comprising or being constituted from one or more proteins that modify cellular regulation, conveniently inserted into vectors for intracellular vehiculation, or potentially comprising or consisting of one or more nucleic acids that directly or indirectly modify (for example by means of the proteins encoding for them or by way of down-regulation) the cellular regulation in the form of mRNA or in suitable vectors, in which this modification contributes to the restoration of differentiated cells and cells derived from them by cellular division into a pluripotent state.

### DETAILED DESCRIPTION OF THE FIGURES

Figure 1 shows the structure of the full-length (FL) transcript of the gene EIF3E3 alias INT6.

Figure 2 shows the structure of the transcript and of the protein EIF3E-5a as hypothesised from the amino acid sequence. Upper half: INT6-5a (or EIF3E-5a) is a transcript of 771 nt comprising the first 5 exons and the first 279 nt of the 5^{th} intron of the human gene INT6. Lower half: Amino acid sequence of the hypothetical human protein EIF3E-5a.

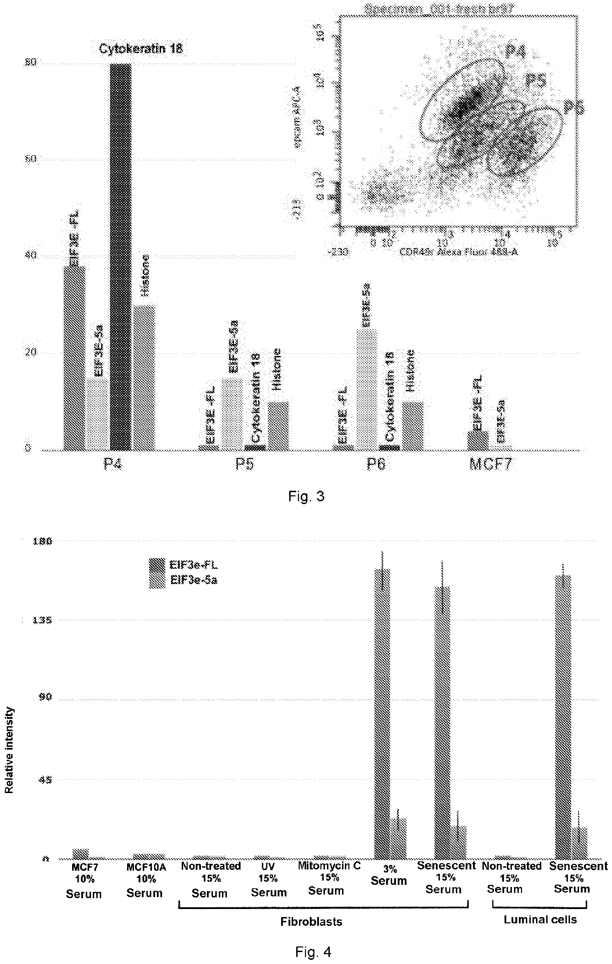

Figure 3 shows the expression of INT6-FL (EIF3E-FL) and INT6-5a (EIF3E-5a) in cells obtained from normal dissociated human mammary tissue, sorted by way of cytofluorometry (FACS) with antibodies EPCAM and CD49f. EIF3E-FL is significantly increased in terminally differentiated or senescent mammary cells (P4 population: high ECAM and low CD49f) compared with basal/stem/progenitor cells (P6 population: low EPCAM and high CD49f) or in immortalised cells (MCF7). Cytokeratin 18 was used as marker for the terminally differentiated mammary luminal cells. High levels of histones suggest that the luminal cells (P4 population) are kept in a more closed chromatin/heterochromatin state compared with cells of the P6 population. The configuration of closed chromatin is normally associated with the cells terminally differentiated into a permanent state of proliferative dormancy and of restricted transcriptional activity. Low levels of histones in the basal-like/stem/progenitor cells (P6 population) suggest that the chromatin in this population is kept in a more open/euchromatin state, normally associated with the undifferentiated cells. The levels of cytokeratin 8 and histones were not measured in the MCF7 cells in this experiment.

Figure 4 shows that the senescent cells express high levels of EIF3E-FL. Since the results of Figure 3 show that the highest level of expression of EIF3E-FL was encountered in terminally differentiated luminal cells or in cells kept in a permanent state of proliferative dormancy, that is to say in cells having a closed configuration of the chromatin, in order to determine whether INT6-FL (EIF3E-FL) is up-regulated as a result of the inhibition of the proliferation or due to the closed confirmation of the chromatin, the level of expression of INT6-FL was assessed in non-differentiated cells deriving from normal mammary tissue (Br95, fibroblasts and luminal cells) cultivated in different culture conditions. Mammary cells from a young patient (fibroblasts and luminal cells) with extensive ability to proliferate and therefore with an open chromatin state were cultivated in 5 different conditions: in medium containing 3 % serum (1) to slow the proliferation and maintain the dormancy; in medium containing 15 % serum (2) to keep the cells at a high rate of proliferation; or treated with UV (3) and with mitomycin-C (4) to inhibit the proliferation. The cells of conditions 3 and 4 were harvested for the analyses of expression of INT6/FL (EIF3E-FL) 72 hours after the treatment with UV and mitomycin C.

In addition, the luminal mammary cells Br95 were cultivated in medium containing 15 % serum (which keeps the cells at a constant rate of high proliferation), but propagated by long-term passaging (for almost 3 months) to induce senescence (5).

The induction of the senescence translated into the loss of the proliferative ability and into the conversion of the conformation of the chromatin from euchromatin to chromatin in a permanently closed state. Treatment with UV and with mitomycin C instead inhibits cell proliferation, leaving the chromatin open in the conformation due to the cross-linking of the DNA that has occurred with the treatment.

The results show that, in senescent mammary cells (luminal or fibroblast), high levels of INT6-FL (EIF3E-FL) were found compared with cells at early passages cultivated in 3 and 15 % serum, compared with MCF7 or MCF10A cell lines that have unlimited proliferation capability and compared with cells treated with UV or mitomycin-C.

The results indicate that high levels of expression of INT6-FL in senescent cells cannot be caused by the induction of damage to the DNA or by the dormancy or by the fact that the cells were cultivated in 15 % serum insofar as cells treated with ultraviolet rays or with mitomycin-C or cultivated with 15 % serum do not express high levels of INT6-FL. The results also suggest that the up-regulation of INT6-FL is associated with a compact conformation of heterochromatin and not with the inhibition of the proliferation induced by the cross-linking.

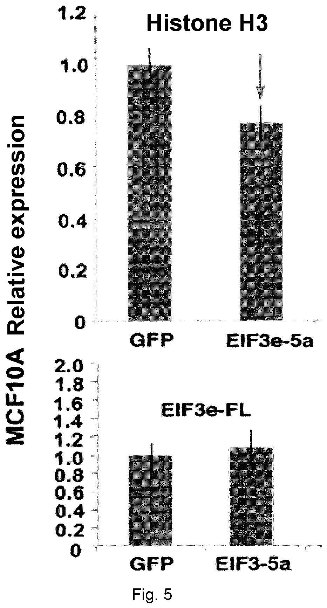

Figure 5 shows the levels of cellular regulation of INT6-5a (EIF3E-5a). Top graph. The up-regulation of INT6-5a in MCF10A cells (MCF10A-5a) promotes the down-regulation of 20 % of the protein levels of the histone H3 compared with the control GFP-c1. Bottom graph. The up-regulation of INT6-5a in MCF10A cells (MCF10A-5a) does not modify the levels of the protein EIF3E-FL compared with the control GFP-c1.

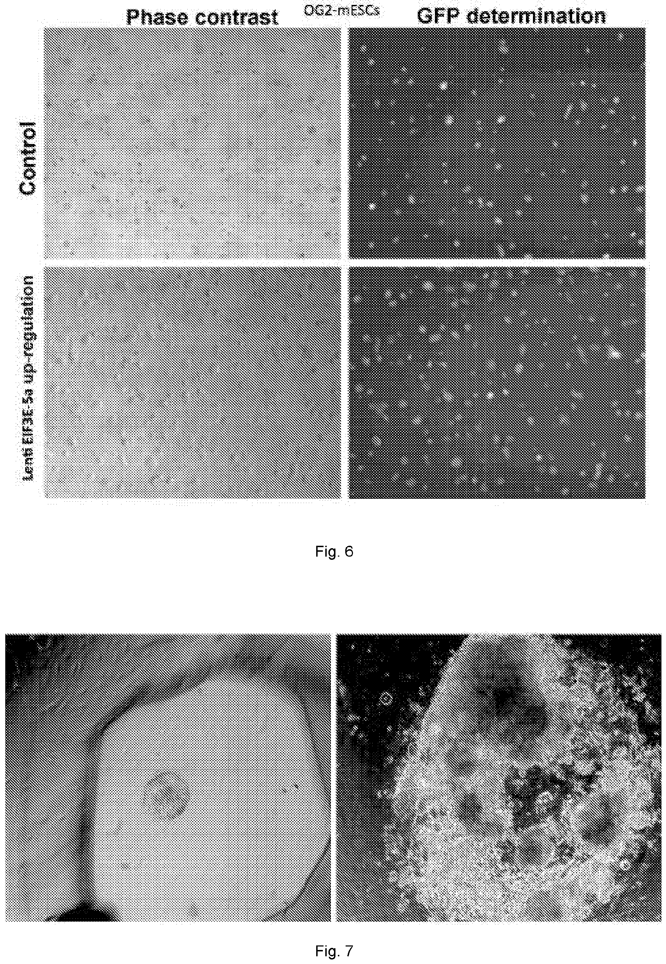

Figure 6 shows the effects of the regulation of INT6-5a. The up-regulation of human mRNA INT6-5a (5-5 in the photo) in pluripotent mouse embryonic stem cells (OG2 mESCs) generates 2-3 times more embryonic stem colonies compared with the control (NT) (light area on the left, FITC fluorescence on the right). The embryonic mouse stem cells OG2 were obtained from transgenic mice generated from embryonic mouse stem cells that express the transgene GFP (green fluorescent protein) under the control of the enhancer of the pluripotency gene OCT-4 as described by Hubner K et al. "Derivation of oocytes from mouse embryonic stem cells." Science. 2003, 300:1251-56.

Figure 7 box to the left: Images representative of iPSCs generated for reprogramming of human neonatal fibroblasts with lentiviral constructs of Oct4, Sox2, Klf4 and c-Myc and (box to the right) with Oct4, Sox2, Klf4 and cMyc with EIF3E-5a. Colonies that are larger and greater in number are present in the condition in which the construct that expresses mRNA EIF3E-5a was added to the reprogramming cocktail, suggesting that EIF3E-5a promotes both cell proliferation and the remodelling of the chromatin to a more permissive state favourable for cellular reprogramming.

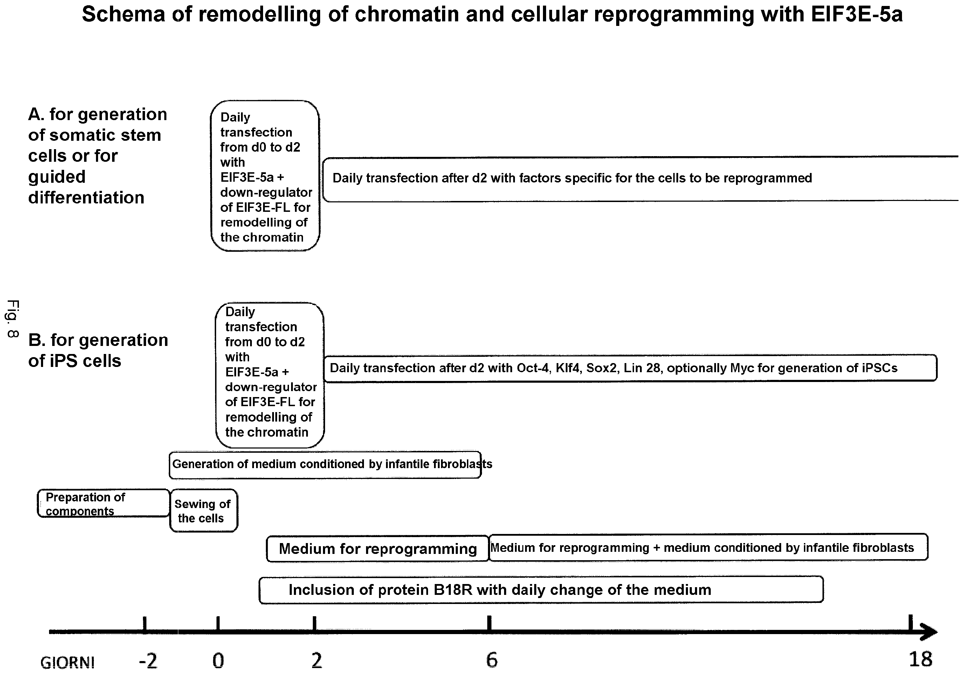

Figure 8: Figure 8 shows a summarising schema of the preferred embodiment of the cellular reprogramming procedure according to the description. Infantile fibroblasts are indicated, but, as is clear from the present description, the procedure can be applied successfully generally also to somatic cells and in particular to senescent somatic cells or to cells taken from elderly individuals.

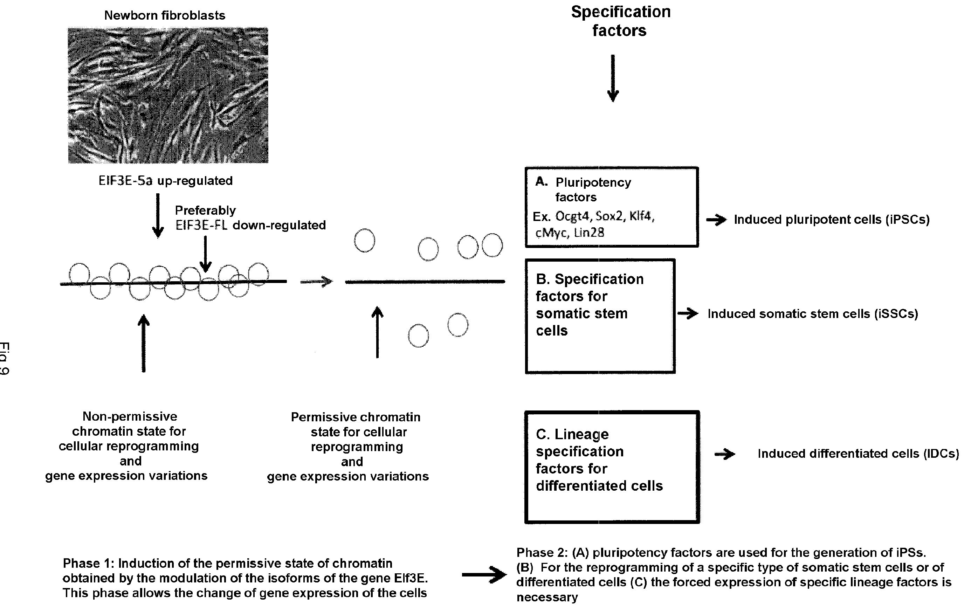

Figure 9: The figure schematically shows a preferred embodiment of the cellular reprogramming protocol by way of repeated transient transfection. Infantile fibroblasts are shown, however, as is clear from the present description, the procedure can be applied successfully generally also to somatic cells and in particular to senescent somatic cells or to cells taken from elderly individuals or in any conditions in which the cells express high levels of EIF3E-FL.

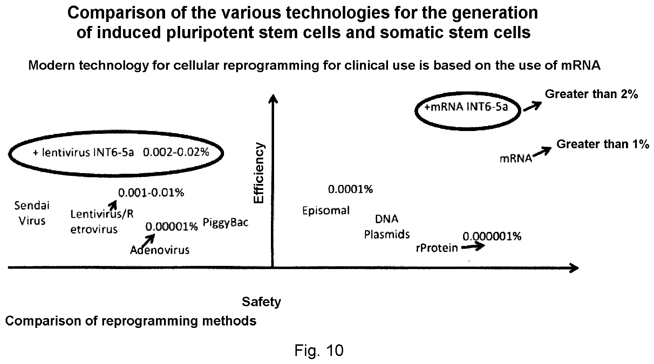

Figure 10 shows data obtained comparing the methods of the description (both with lentivirus and with transfection) compared with conventional methods.

The data obtained shows that the reprogramming based on the use of mRNA is the quickest, safest and most efficient method for the generation of clinically acceptable cells with genomes free from viral insertions or modifications linked with the use of viral vectors.

The use of lentivirus/retrovirus/adenovirus and episomal mini-rings generates insertional mutagenesis, and systems that are based on the use of recombinant proteins have an extremely low efficiency.

The data shown in the figures shows that the reprogramming based on mRNA, in particular with EIF3E-5a according to the description, is the most efficient and safest method most suitable for the generation of iPS for clinical uses.

The data shows that even with conventional methods, which use lentivirus, the protocol of the description, which uses EIF3E-5a, is more effective in any case.

Modified by Miyazaki et al. Emerging Methods for Preparing iPS Cells,Jpn.J.Clin. Oncol.(2012) 42, 773-779

### DETAILED DESCRIPTION OF THE SEQUENCES

SEQ ID NO 1 nucleotide sequence EIF3E-5a (cDNA) : gagcacagac tcccttttct ttggcaagat ggcggagtac gacttgacta ctcgcatcgc gcactttttg gatcggcatc tagtctttcc gcttcttgaa tttctctctg taaaggagat atataatgaa aaggaattat tacaaggtaa attggacctt cttagtgata ccaacatggt agactttgct atggatgtat acaaaaacct ttattctgat gatattcctc atgctttgag agagaaaaga accacagtgg ttgcacaact gaaacagctt caggcagaaa cagaaccaat tgtgaagatg tttgaagatc cagaaactac aaggcaaatg cagtcaacca gggatggtag gatgctcttt gactacctgg cggacaagca tggttttagg caggaatatt tagatacact ctacagatat gcaaaattcc agtacgaatg tgggaattac tcaggagcag cagaatatct ttattttttt agagtgctga tggagtcttg ctatgttgcc caggctgatc tcaaactcct ggcctgaagt gatcctccct cctcagcctt tcaatgcttt gggatttaca gccatgagcc tctgcaccct gcctgtctaa gatttctcta gttaatccta gaagttggaa aaagacctta aaacagagac tttttgtctt ccaaccttgg aagagtttgt ttctttttgc ctttactttc tggtgctttt ttcttcactt gtagctccac atgttgcaaa ataaagtctc atggctgctg tcaaaaaaaa aaaaaaaaaa aaaaaaaaaa aa
   when this sequence must represent an RNA molecule (ex m RNA) each symbol "t" shown above is to be considered as substituted by the symbol "u" as shown below in
   SEQ ID NO 19 nucleotide sequence EIF3E-5a (RNA) : gagcacagac ucccuuuucu uuggcaagau ggcggaguac gacuugacua cucgcaucgc gcacuuuuug gaucggcauc uagucuuucc gcuucuugaa uuucucucug uaaaggagau auauaaugaa aaggaauuau uacaagguaa auuggaccuu cuuagugaua ccaacauggu agacuuugcu auggauguau acaaaaaccu uuauucugau gauauuccuc augcuuugag agagaaaaga accacagugg uugcacaacu gaaacagcuu caggcagaaa cagaaccaau ugugaagaug uuugaagauc cagaaacuac aaggcaaaug cagucaacca gggaugguag gaugcucuuu gacuaccugg cggacaagca ugguuuuagg caggaauauu uagauacacu cuacagauau gcaaaauucc aguacgaaug ugggaauuac ucaggagcag cagaauaucu uuauuuuuuu agagugcuga uggagucuug cuauguugcc caggcugauc ucaaacuccu ggccugaagu gauccucccu ccucagccuu ucaaugcuuu gggauuuaca gccaugagcc ucugcacccu gccugucuaa gauuucucua guuaauccua gaaguuggaa aaagaccuua aaacagagac uuuuugucuu ccaaccuugg aagaguuugu uucuuuuugc cuuuacuuuc uggugcuuuu uucuucacuu guagcuccac auguugcaaa auaaagucuc auggcugcug ucaaaaaaaa aaaaaaaaaa aaaaaaaaaa aa
SEQ ID NO 2 amino acid sequence EIF3E-5a: Met Ala Glu Tyr Asp Leu Thr Thr Arg Ile Ala His Phe Leu Asp Arg His Leu Val Phe Pro Leu Leu Glu Phe Leu Ser Val Lys Glu lie Tyr Asn Glu Lys Glu Leu Leu Gln Gly Lys Leu Asp Leu Leu Ser Asp Thr Asn Met Val Asp Phe Ala Met Asp Val Tyr Lys Asn Leu Tyr Ser Asp Asp lie Pro His Ala Leu Arg Glu Lys Arg Thr Thr Val Val Ala Gln Leu Lys Gln Leu Gln Ala Glu Thr Glu Pro lie Val Lys Met Phe Glu Asp Pro Glu Thr Thr Arg Gln Met Gln Ser Thr Arg Asp Gly Arg Met Leu Phe Asp Tyr Leu Ala Asp Lys His Gly Phe Arg Gln Glu Tyr Leu Asp Thr Leu Tyr Arg Tyr Ala Lys Phe Gln Tyr Glu Cys Gly Asn Tyr Ser Gly Ala Ala Glu Tyr Leu Tyr Phe Phe Arg Val Leu Met Glu Ser Cys Tyr Val Ala Gln Ala Asp Leu Lys Leu Leu Ala
SEQ ID NO 3: EIF3E-5a sense primer
   gcatggtttt aggcaggaat attt 24
SEQ ID NO 4: EIF3E-5a antisense primer
   cttcaggcca ggagtttgag a 21
SEQ ID NO 5: EIF3E (NM_001568) sense primer
   cagatgttgg ccatgaatat tga 23
SEQ ID NO 6: EIF3E (NM_001568) antisense primer
   gcccagttag gagcctctga 20
SEQ ID NO 7: GAPDH (NM_002046) sense primer
   gcaccgtcaa ggctgagaac 20
SEQ ID NO 8: GAPDH (NM_002046) antisense primer
   agggatctcg ctcctggaa 19
SEQ ID NO 9: HPRT1 (NM_000194) sense primer
   tttgctgacc tgctggatta ca 22
SEQ ID NO10: HPRT1 (NM_000194) antisense primer
   ggtcattaca atagctcttc agtctgat 28
SEQ ID NO 11: Rpl32 (NM_000994) sense primer
   ttcatccggc accagtca 18
SEQ ID NO 12: Rpl32 (NM_000994) antisense primer
   gtcaatgcct ctgggtttcc 20
SEQ ID NO 13: Rpl34 (NM_000995) sense primer
   tttgacatac cgacgtaggc ttt 23
SEQ ID NO 14: Rpl34 (NM_000995) antisense primer
   gggttcggga cagcctagtt 20
SEQ ID NO 15: sense primer to generate RNA EIF3E-5a
   gactcccttt tctttggcaa gat 23
SEQ ID NO 16: antisense primer to generate RNA EIF3E-5a
   aggtcttttt ccaacttcta ggat 24
SEQ ID NO 17: commercial oligomer for specific inhibition of EIF3E Full Length
   guuuauaugu uaacuuuga 19
SEQ ID NO 18: commercial oligomer for specific inhibition of EIF3E Full Length
   caguguauca gcauuaaca 19

### DETAILED DESCRIPTION OF THE INVENTION

The present invention relates to a nucleic acid molecule as shown in the nucleotide sequence SEQ ID NO 1 or 19 encoding an isoform of the human protein EIF3E that does not comprise exons 6-13, and to a new protein having SEQ ID NO 2 in which said protein is an isoform of the protein EIF3E.

The protein having SEQ ID NO 2 is produced from a new transcript not yet described in the literature, referred to here as EIF3E-5a. Such a transcript is an isoform of the human transcript known in the literature as EIF3E full length (also referred to here as EIF3E-FL) encoded by the gene EIF3E (gene ID 3646, mRNA NM_001568.2), said isoform being encoded by a nucleic acid molecule having nucleotide sequence SEQ ID NO 1 or 19 (also referred to here as EIF3E-5a) or by other nucleic acid molecules having sequences deducible from the amino acid sequence SEQ ID NO 2. The transcript according to the invention thus has the amino acid sequence SEQ ID NO2 deduced from the nucleotide sequence SEQ ID NO 1, and it is reported here for the first time that such sequences can be used with success, in combination with other genes, in the cellular reprogramming of human somatic cells and of somatic cells of other mammals.

The sequence of cDNA encoding the isoform of the invention (SEQ ID NO 1) and the putative sequence of the protein derived from it (SEQ ID NO 2) were filed in confidential form by the authors of the present invention at GenBank on 13 December 2012 and will be made available to the public only on 31 December 2014. As reported in the experimental part, the authors of the present invention have discovered that the up-regulation of the expression of mRNA encoding the protein having SEQ ID NO 2, in transient or stable form, induces an increase in cell proliferation in human and non-human mammalian cells and alters the content of the histones and of the nucleosomes and the state of the chromatin, inducing a euchromatic state (open chromatin) in a manner dependent on the analysed mammalian species.

In accordance with the present description, protein "having" SEQ ID NO 2 means a protein of which the sequence is represented in SEQ ID NO 2 and nucleic acid "having" SEQ ID NO 1 means a nucleic acid molecule of which the sequence is represented in SEQ ID NO 1. It is understood that throughout the present description, when this molecule is a ribonucleic acid, each letter T shown in SEQ ID NO 1 is to be substituted by the letter U. However, when reference is made to RNA or mRNA having SEQ ID NO 1, each letter T in SEQ ID NO 1 is to be considered as substituted by the letter U as normally occurs in RNA (thus, when reference is made in the text to RNA having the sequence SEQ ID NO 1, this sequence corresponds to SEQ ID NO 19).

The induction of cell proliferation and the change in the content of the histones are essential steps for obtaining local and global changes in the structure of the chromatin and changes in the gene expression, these being essential changes for cellular reprogramming.

The authors of the invention have also found that the expression of EIF3E-5a (SEQ ID NO 1 or nucleotide sequences encoding for the transcript having SEQ ID NO 2) is independent of the expression of EIF3E full length.

In this patent application the use of the human transcript INT6-5a (EIF3E-5a) to induce a rise of cell proliferation and remodelling of the chromatin into fibroblasts or other types of cells isolated from human or mammalian tissues is described for the purpose of promoting the generation of somatic stem cells and induced pluripotent stem cells.

The inventors thus propose the use of EIF3E-5a (nucleic acid molecule represented by SEQ ID NO 1 or SEQ ID NO 19 or by nucleotide sequences encoding for the transcript having SEQ ID NO 2) or of the protein of which the amino acid sequence is represented in SEQ ID NO 2 as component of an agent for the production of IPSC cells.

The invention thus also relates to an agent for the production or optimisation of the production of IPSC cells from human or non-human mammalian somatic cells comprising the protein having the sequence SEQ ID NO 2 or a nucleic acid molecule having the sequence SEQ ID NO 1 or SEQ ID NO 19 or a nucleic acid encoding the protein having SEQ ID NO 2.

Since the protein having SEQ ID NO 2 is described here, it is considered to be a standard step for a person of normal knowledge in the relevant technical field to be able to generate nucleotide sequences encoding such a protein on the basis of the genetic code.

If it is therefore considered that the definition "nucleic acid encoding the protein having the sequence SEQ ID NO 2" is sufficiently described in the present description providing SEQ ID NO 2.

The authors of the present invention have also discovered that the down-regulation of the protein EIF3E full length, obtainable for example by way of down-regulation of the mRNA thereof or of the protein encoded from it by means of siRNA (or shRNA exclusively for research purposes) or also by way of inhibition thereof with antibodies or antibody fragments, or by way of microRNA is associated with the immortalisation of the cells and can promote the reversal of cellular senescence.

The authors have in fact found that high levels of INT6-FL (EIF3E-FL) are associated with terminally differentiated or senescent cells (EPCAM high CD49f low or infant foreskin cells) derived from normal tissue (Table 1 below, Figure 3) whereas low levels of INT6-FL are associated with cells derived from tumour tissue (Table 1) and with cellular lines whether tumorigenic (MCF7) or non-tumorigenic (MCF10A). The data obtained suggests that the function of INT6-FL is that of keeping the cells in a closed conformation of the chromatin (temporary or permanent) with limited rise of isotonic proteins and nucleosomes.

**Table 1**

| **Tumour samples** | EIF3-5a | EIF3-FL | **Normal samples** | EIF3-5a | EIF3-FL | **Primary cells isolated from normal mammary tissue** | EIF3-5a | EIF3-FL |
|---|---|---|---|---|---|---|---|---|
| **Patient no. 21** | 52 | 7040 | **Patient no. 21** | not available | not available | BR15 15% serum | 53 | 1966 |
| **Patient no. 27** | 23 | 5205 | **Patient no. 27** | 35 | 18775 | Br no. 15 10% serum | 28 | 2860 |
| **Patient no. 28** | 66 | 7429 | **Patient no. 28** | 190 | 17845 | Br no. 5 15% serum | 37 | 1421 |
| **Patient no. 29** | 37 | 2106 | **Patient no. 29** | 108 | 11615 | Br no. 5 10% serum | 14 | 1692 |
| **Patient no. 30** | 45 | 2054 | **Patient no. 30** | not available | not available | Br no. 83 15% serum | 50 | 1493 |
| **Patient no. 31** | 17 | 1015 | **Patient no. 31** | 86 | 11608 | Br no 83 10% serum | 34 | 1590 |
| **Patient no. 32** | 13 | 2294 | **Patient no. 32** | not available | not available | Br no. 83 late passage | 74 | 2671 |
| | | | | | | | | |
| **Tumorigenic mammary cell line** | | | **Tumorigenic mammary cell line** | | | | | |
| **MCF7** | 26 | 1645 | **MCF10A** | 43 | 1060 | | | |

**Table 1.** The levels of expression of mRNA INT6-FL and INT6-5a (EIF3E-FL and EIF3E-5a) were assessed in normal and tumoral tissues *in toto* (patients 21-32), in cell lines (MCF7 and MACF10A) and in cells isolated from normal mammary tissue (patients Br5, Br15 and Br83) cultivated for 2 weeks with different serum concentrations (10%) and (15%) to modulate the proliferation, or propagated in the long term in culture to induce senescence (see Br83 with late passages). In the normal mammary tissue INT6-FL there was significantly more expression (on average 15,000 copies per cell) compared with the tumoral mammary tissue (on average 4000 copies per cell). In mammary cell lines MCF7 and MACF10A, INT6-FL was expressed to a significantly lower extent (1600 and 1000 copies per cell respectively).
Cells at early stages propagated in medium with lowest concentration of serum (10%) or the cells cultivated in the long term (Br83 late passages) demonstrated higher levels of expression of INT6-FL compared with cells with early passages propagated in medium with high serum concentration (15%). Higher levels of expression of INT6-FL suggest that INT6-FL induces or is associated with the state of proliferative dormancy of the cells cultivated in conditions that do not favour proliferation or with the state of proliferative interruption associated with the onset of senescence in terminally differentiated cells.

It is known that the closed confirmation of the chromatin is associated with a state of proliferative dormancy and has the purpose of hindering proliferation and avoiding changes in gene expression. The authors of the present invention have in fact observed that the reduction of INT6-FL (EIF3E-FL) in cells kept in a transient or stable conformation of heterochromatin induces a more open state of the chromatin, which allows the cells to regain the ability the proliferate and to remodel the gene expression. The gaining of the ability to proliferate and to remodel the gene expression is essential for cellular reprogramming and is essential to induce trans-differentiation, i.e. the generation of iPSC cells or of somatic stem cells.

The sustained induction of a state of open chromatin following the loss or down-regulation of INT6-FL may lead to the immortalisation of the cells.

The open chromatin in addition may allow, but not induce directly, the dedifferentiation of the cells. The loss or down-regulation of INT6-FL is associated with the immortalisation of the cells and may promote the reversal of the cellular senescence. On the basis of obtained data, the authors thus hypothesize that the up-regulation of INT6-FL should induce the cells into a state of proliferative dormancy.

The authors of the invention have also observed that high levels of EIF3E-5a (or up-regulation thereof) promote the onset of proliferation (or induce proliferation in cells that already have proliferative capability) and the reduction of the isotonic proteins of chromatin. The onset of cell proliferation and the opening of the chromatin state are essential to allow variations in gene expression, these being necessary to induce trans-differentiation, cellular reprogramming and the generation of iPSC cells or of adult stem cells.

The results obtained by the authors have made it possible to further optimise the efficiency in the induction of the opening of the chromatin and cell proliferation by reprogramming and to conclude that the induction of trans-differentiation and the generation of iPSC cells, or the generation of somatic stem cells are obtained more efficiently with the concomitant or simultaneous down-regulation of INT6-FL and the up-regulation of INT6-5a.

In particular, it should be noted that the current technologies for cell programming are more effective if cells from young patients are used and that there is instead a great need for new and effective technologies and agents for the generation of iPSC cells for elderly patients.

The technologies currently existing for cellular reprogramming are more effective if cells obtained from young patients are used, and there is thus currently a great need for innovative and effective technologies for the generation of iPCSs from elderly patients. Young patients require substitution of the stem cells due to chronic diseases or accidental lesions, whereas elderly patients require substitution of the stem cells due to the loss of the capability of regeneration of organs or tissues caused by the natural ageing process. Cells of elderly patients demonstrate a lower efficiency of cellular reprogramming and a low efficiency in the generation of iPSs due to the fact that these cells have reached a permanent state of senescence.

Since the down-regulation of INT6-FL is necessary in order to cancel the state of senescence, in one embodiment of the present invention the up-regulation of EIF3E-5a and the down-regulation of EIF3E-FL or of mRNAs that encode it are combined in order to maximise the reprogramming efficacy, above all in the case of senescent cells or cells isolated from elderly patients.

In one embodiment the agent for the production and for the optimisation of the production of IPSC cells from human or non-human mammalian somatic cells of the invention thus also comprises a specific down-regulator of EIF3E Full Length that does not interfere with the regulation of nucleic acid molecules having SEQ ID NO 1 or SEQ ID NO 19 and/or of proteins having SEQ ID NO 2 as defined in the claims. Such a reagent could be, for example, a nucleic acid selected from an siRNA, an shRNA, an antibody against the protein having SEQ ID NO 2 or a microRNA having EIF3E-FL as target.

Down-regulators specific for EIF3E-FL which do not influence the regulation of EIF3E-5a are easily identifiable by a person skilled in the art since it is sufficient that they have as target any region of the nucleotide sequence or of the amino acid sequence corresponding to exons from 6 to 13 of EIF3E-FL (gene or protein), which are absent from SEQ ID NO 1 or 19, and 2.

The agent according to the invention will also comprise other molecules with amino acid or protein sequences selected from one or more transcription factors normally expressed in cells and/or factors normally expressed in embryonic stem cells and/or growth factors or factors linked to tumours. The additional selected factors may be one or more of those known in the literature (see references above) already used for cellular reprogramming.

An example of such factors suitable for realising the present invention is indicated specifically on pages 32 and 33 of patent application WO2014/065435, these including, but not being limited to:
(1) Oct3/4, Klf4, c-Myc
(2) Oct3/4, Klf4, c-Myc, Sox2 (Sox2 can be substituted with SoxI, Sox3, SoxIS, Sox17 or Sox18); Klf4 can be substituted with Klfl, Klf2 or KlfS; c-Myc can be substituted with T58A (active mutant), N-Myc, or L-Myc)
(3) Oct3/4, Klf4, c-Myc, Sox2, Fbx15, Nanog, Eras, ECAT15-2, Tcll, β catenin (active mutant S33Y)
(4) Oct3/4, Klf4, c-Myc, Sox2, TERT, SV40 Large T antigen (SV40LT), (S) Oct3/4∼ Klf4, c-Myc, Sox2, TERT, HPV16 E6
(6) Oct3/4, Klf4, c-Myc, Sox2, TERT, HPV16 E7
(7) Oct3/4, Klf4, c-Myc, Sox2, TERT, HPV6 E6, HPV16 E7
(8) Oct3/4, Klf4, c-Myc, Sox2, TERT, Bmil etc.

In one embodiment the agent for the production and for the optimisation of the production of IPSCs from human or non-human mammalian somatic cells of the invention will comprise the human transcription factor SOX-2, the human Krueppel-like 4 factor, the POU domain, class 5, transcription factor 1 and LIN28 or nucleic acid molecules encoding them.

According to the invention the proteins SOX2, KLF4, OCT4 and LIN28 and the nucleic acid molecules encoding them will have the sequences such as those available in the databases specified below and isoforms thereof having the same functions. The data available in the uniprot databases is specified below.

P48431- SOX2_HUMAN Protein name: Transcription factor SOX-2; Gene name: SOX2; Gene ID: 6657, updated on 5 October 2014; Official gene symbol: SOX2; official full gene name: SRY (sex determining region Y)-box 2; RefSeq of the RNA: NM_003106.3, 2520 bp mRNA linear PRI updated on 5 May 2014; RefSeq_of the protein: NP_003097.1, 317 aa linear, PRI last updated on 5 May 2014.

O43474- KLF4_HUMAN Protein name: Krueppel-like factor 4; Gene names: KLF4, EZF, GKLF; Gene ID: 9314, last updated on 5 October 2014; Official gene symbol: KLF4; official full gene name: Krueppel-like factor 4 (good); RefSeq_of the RNA: NM_004235.4 2949 bp mRNA linear PRI last updated on 25 May 2014; RefSeq_of the protein: NP_004226.3 479 aa linear PRI last updated on 25 May 2014
Q01860- PO5F1_HUMAN Protein name: POU domain, class 5, transcription factor 1; Gene names: POU5F1, OCT3, OCT4, OTF3; Gene ID: 5460, last updated on 5 October 2014; Official gene symbol: POU5F1; Official gene name: POU class 5 homeobox 1; RefSeq_of the RNA: NM_001173531.2, 1589 bp mRNA linear PRI last updated on 4 May 2014; RefSeq_of the protein: NP_001167002 190 aa, linear PRI last updated on 4 May 2014.
LIN28
Gene ID: 79727; last updated 8 October 2014; Official symbol: LIN28A; official full gene name: lin-28 homolog A (C. elegans); RefSeq_of the RNA: NM_024674.4, 4014 bp mRNA linear PRI last updated on 25 May 2014; RefSeq_of the protein: NP_078950.1, 209 aa linear PRI last updated on 25 May 2014.

In one embodiment of the invention the agent for cellular reprogramming as described here therefore does not comprise factors linked to tumours.

In accordance with a further embodiment the agent of the invention may also comprise the Myc proto-oncogene protein or a nucleic acid encoding it. Such protein is available in the databases, and is specified in uniprot as P01106- MYC_HUMAN Protein name: Myc proto-oncogene protein; Gene names: MYC, BHLHE3 Gene ID: 4609, last updated on 5 October 2014; Official gene symbol: MYC; official full gene name: v-myc avian myelocytomatosis viral oncogene homolog; RefSeq_of the RNA: NM_002467.4, 2379 bp mRNA linear PRI last updated on 25 May 2014; RefSeq_of the protein: NP_002458.2 454 aa linear PRI last updated on 25 May 2014

In accordance with the invention the agent could contain an agent that regulates negatively (down-regulator) and in a specific way (i.e. does not interfere with the expression of the activity of the other proteins specified above) EIF3E Full Length as defined in the claims. With regard to the down-regulator specific for EIF3E Full Length, this could be in any one of the above-specified forms.

In accordance with one embodiment of the invention, the nucleic acids can be inserted into a lentiviral vector or into a non-viral vector as taught in detail in the materials and methods of the following publication and in the description detailed below.

Takahashi K, Tanabe K, Ohnuki M, Narita M, Ichisaka T, Tomoda K, Yamanaka S. "Induction of pluripotent stem cells from adult human fibroblasts by defined factors". Cell. 2007 Nov 30;131(5):861-72.

In particular for the preparation of the cells, as reported on page 869 in the left-hand column halfway down the page to the right-hand column halfway down the page; whereas, for the production of viral and viral infection particles, as reported on page 869 from line 34 to line 64 in the right-hand column and on page 870, the first 3 lines at the top of the left-hand column.

The vectors for the generation of the lentiviral particles used in the method described by Yamanaka (above) in addition to the lentiviral vector prepared under point 5 are available as polycistronic constructs from Addgene (Plasmid repository) obtained in accordance with the protocol described in Carey B.W., Markoulaki S., Hanna J., Saha K., Gao O., Mitalipova M., Rudolf Jaenisch R. Reprogramming of murine and human somatic cells using a single polycistronic vector PNAS_2009 _ vol. 106_no. 1_157-162.

Vector analogues comprising SEQ ID 1 or other sequences encoding SEQ ID 2 can be produced in accordance with methods known to a person skilled in the art by inserting the aforementioned nucleotide sequences into lentiviral or non-viral vectors (even if the non-viral constructs have low efficiency and are therefore less advantageous) such as those used in the above references or similar vectors available commercially in accordance with the manufacturer's instructions or available in the literature.

The description therefore relates to lentiviral or non-viral vectors that express one or more of the above-listed proteins, including EIF3E-5a, systems of such vectors, and use thereof for cellular reprogramming.

Since the use of retroviral or lentiviral vectors leads to the insertion of foreign sequences in the genome, in an uncontrollable manner, the authors of the present invention have also developed reprogramming methods based on the transient up-regulation of mRNA (recombinant, produced artificially) encoding the above-listed proteins since these are considered to be safest for generating iPSC cells, for clinical applications. In addition, since c-MYC is a well-established oncogene, research is heavily focused on the identification of new compounds, small molecules, genes or micro-RNA that could functionally substitute c-MYC in the generation of iPSC cells. In addition, since c-MYCs promote local and global chromosomal changes that are not yet well characterised, this makes the iPSC cells problematic for use in patients due to the possible induction of malignant transformation.

The preferred approach for cellular reprogramming for clinical-type purpose proposed by the authors of the present invention is based exclusively on the use of conventional transient transduction methods, but performed with mRNA for up-regulation or synthetic oligonucleotides for down-regulation of the above-specified proteins or mRNA thereof. One of the strategies for generating human or mammalian iPSC cells according to the invention, however, uses transient transfection of mRNA having SEQ ID NO 19, which corresponds to SEQ ID NO 1 in that each letter "t" is substituted by the letter "u" where, in accordance with conventional systems, "t" stands for thymidine and "u" stands for uracil.

Such technology may comprise the use of mRNA (recombinant, artificial) of the above-specified genes, such as Sox2, Klf4 and POU5f1 (Oct3/4), in the quantities specified in the literature and optionally stoichiometric levels of mRNA of c-MYC reduced by 50-70 % compared with the levels reported in the literature.

In one embodiment the agent of the invention will comprise mRNA having SEQ ID NO 19 or having other nucleotide sequences which can be translated from the cell encoding a protein having SEQ ID NO 2.

In accordance with the invention said agent will also comprise one or more sequences of mRNA encoding one or more of the above-specified proteins, said sequences being available in the public databases, such as GenBank. In a particular embodiment said agent will comprise mRNA having SEQ ID NO 19 or other sequences able to be translated from the cell encoding a protein having SEQ ID NO 2 in combination with mRNA encoding the human transcription factor SOX-2, the human factor Krueppel-like 4, the POU domain, class 5, transcription factor 1 and LIN28. Such MRNAs can be obtained commercially or in a synthesised manner in laboratory from IVT constructs in accordance with the protocol described by Warren et al 2010, (page 9, sole column, lines 17 to 35 from the nucleotide sequences available in the databases indicated below. The data available in the uniprot database is specified below.

P48431- SOX2_HUMAN Protein name: Transcription factor SOX-2; Gene name: SOX2 ; Gene ID: 6657, updated on 5 October 2014; Official gene symbol: SOX2; official full gene name: SRY (sex determining region Y)-box 2; RefSeq of the RNA: NM_003106.3, 2520 bp mRNA linear PRI updated on 5 May 2014; RefSeq_of the protein: NP_003097.1, 317 aa linear, PRI last updated on 5 May 2014.

043474- KLF4_HUMAN Protein name: Krueppel-like factor 4; Gene names: KLF4, EZF, GKLF; Gene ID: 9314, last updated on 5 October 2014; Official gene symbol: KLF4; official full gene name: Krueppel-like factor 4 (gut); RefSeq_of the RNA: NM_004235.4 2949 bp mRNA linear PRI last updated on 25-May-2014; RefSeq_of the protein: NP_004226.3 479 aa linear PRI last updated on 25 May 2014
Q01860- PO5F1_HUMAN Protein name: POU domain, class 5, transcription factor 1; Gene names: POU5F1, OCT3, OCT4, OTF3; Gene ID: 5460, last updated on 5 October 2014; Official gene symbol: POU5F1; Official gene name: POU class 5 homeobox 1; RefSeq_of the RNA: NM_001173531.2, 1589 bp mRNA linear PRI last updated on 4 May 2014; RefSeq_of the protein: NP_001167002 190 aa, linear PRI last updated on 04-May-2014.
LIN28
Gene ID: 79727 last updated on 8 October 2014; Official symbol: LIN28A; official full gene name: lin-28 homolog A (C. elegans); RefSeq_of the RNA: NM_024674.4, 4014 bp mRNA linear PRI last updated on 25 May 2014; RefSeq_of the protein: NP_078950.1, 209 aa linear PRI last updated on 25 May 2014

In one embodiment of the invention the agent for cellular reprogramming as described here therefore does not comprise factors linked to tumours.

In accordance with a further embodiment, the agent of the invention may also comprise the Myc proto-oncogene protein or a nucleic acid that encodes it. Such a protein is available in the databases, and is specified in uniprot as P01106-MYC_HUMAN Protein name: Myc proto-oncogene protein; Gene names: MYC, BHLHE3 Gene ID: 4609, last updated on 5 October 2014; Official gene symbol: MYC; official full gene name: v-myc avian myelocytomatosis viral oncogene homolog; RefSeq_of the RNA: NM_002467.4, 2379 bp mRNA linear PRI last updated on 25 May 2014; RefSeq_of the protein: NP_002458.2 454 aa linear PRI last updated on 25 May 2014.

For the purposes of realising the present invention, the proteins (recombinant) encoded by the mRNAs specified above can be used as agent for cellular reprogramming as described above. Such proteins can be induced in the cell in accordance with standard protocols known in the literature, many of said protocols being summarised for example in Claire O. Weill, Stéphanie Biri, Abdennaji Adib, and Patrick Erbacher "A practical approach for intracellular protein delivery" Cytotechnology. Jan 2008; 56(1): 41-48. Published online Oct 16, 2007. DOI: 10.1007/s10616-007-9102-3 or also in Iwan Walev, Sebastian Chakrit Bhakdi, Fred Hofmann, Nabil Djonder, Angela Valeva, Klaus Aktories and Sucharit Bhakdi "Delivery of proteins into living cells by reversible membrane permeabilization with streptolysin-O" PNAS March 13, 2001 vol. 98 no. 6 3185-3190. A person skilled in the art will be able to apply such known protocols by inserting into the cells the proteins encoded by the above-specified mRNAs. In particular, the research conducted by the authors of the present invention shows that the transient expression of INT6-5a does not convert healthy cells into tumour cells and does not promote the induction of tumours when non-tumour cells transduced with INT6-5a are transplanted into nude mice. INT6-5a therefore does not act as an oncogene. It has been observed that the increase of cell proliferation is only temporary when the expression of INT6-5a is transient. It was also observed that the transient remodelling of the chromatin did not produce, in long-term cultures, a rise in the rate of mutations of the normal primary cells.

For this reason the agent as described above may advantageously also comprise a reagent that down-regulates the expression of INT6 full length in a specific way (without interfering with the level of expression of INT6-5a (encoded by SEQ ID NO 1 or 19)).

Such a reagent may be, for example, a nucleic acid selected from an siRNA, an shRNA, or an antibody or a microRNA that acts in a specific way on the full length form (exons 1-13) of EIF3E as already defined above. In the preferred embodiment, the generation of IPSC cells was implemented by way of repeated transient transfection of mammalian somatic cells, including human cells.

The transfection procedure can be performed in accordance with the schema indicated in Figure 8.

To summarise, the procedure provides a daily transfection from day 0 to at least day 18 with EIFE-5a mRNA (having SEQ ID NO 1 in which T becomes U, that is to say SEQ ID NO 19 or in any case an mRNA encoding for SEQ ID NO 2) and a daily transfection, from day 2 to at least day 18, with one or more mRNAs encoding the human transcription factor SOX-2, the human factor Krueppel-like 4, the POU domain, class 5, transcription factor 1, LIN28 and, optionally, c-Myc. The RNAs for the cellular transfection can be provided using recombinant expression systems or can be organised and generated artificially by companies that provide such services commercially. The generation of the RNAs is routine, the sequences of interest being available to the public and the sequence EIF3E-5a being made available. In any case, for the generation of RNAs suitable for transfection relative to EIF3E-5a, the primers provided here as SEQ ID 15 and 16 can be used. The protocol detailed for the preparation of the mRNAs is illustrated in Figure S1 and is described in the key of Figure S1, specified in the Additional Information of the article by Warren et al. Highly efficient reprogramming to pluripotency and directed differentiation of human cells with synthetic modified mRNA. Cell Stem Cell. 2010, 7:618-630

In one embodiment the transfection on day 2 will be carried out with mRNAs encoding the human transcription factor SOX-2, the human factor Krueppel-like 4, the transcription factor POU, class 5, transcription factor 1 and LIN28. In the embodiments described above and below, the factor cMyc can be used optionally. Other factors known in the literature as factors suitable for the realisation of cellular reprogramming protocols are not excluded from the present description.

In addition, in a preferred embodiment, the protocol will comprise daily transfection from day 0 to at least day 18 with an agent for the selective down-regulation of EIF3E FL (down-regulation of EIF3E FL) as defined above.

This last embodiment considerably increases the efficacy of reprogramming when compared with conventional protocols and increases in particular the efficacy of reprogramming of senescent somatic cells or somatic cells taken from elderly individuals.

The protocol can therefore provide the following steps defined schematically: Generation of the iPS cells
Human or mouse fibroblasts are sewn and cultivated for the purpose of harvesting the semi-confluence.

The generation of iPSCs comprises the transfection with a cocktail of genes including mRNA of the isoform EIF3E-5a and sRNA against the isoform EIF3E FL every 24 hours for the first two days in order to convert the heterochromatin to a permissive chromatin state, followed by transfection repeated every 24 hours for 18 days, with a cocktail that includes mRNA of Oct-4, Sox2, Klf4, cMyc, GFP and optionally LIN28, in molar stoichiometry of 3: 1: 1: 1: 1: 1 (:1).

The present invention also provides a kit for preparing human or non-human mammalian induced pluripotent stem cells including one or more aliquots of the reagents as defined above.

The reagents may be in the form of proteins, of proteins inserted into suitable vectors for intracellular vehiculation, or in the form of nucleic acids, inserted into vectors or also in a form suitable for use in transfection protocols (RNA).

In particular, the kit may be a kit for improving the efficiency of existing reprogramming protocols and may comprise aliquots of up-regulators of EIF3E-5a (for example expression vectors comprising SEQ ID NO 1 or 19 or sequences encoding SEQ ID NO 2, RNA having SEQ ID NO 19 or of which the sequence in any case encodes for SEQ ID NO 2, proteins having SEQ ID NO 2 optionally inserted into vectors suitable for intracellular vehiculation) and aliquots of down-regulators specific for EIF3E full length (that is to say which do not interfere with the expression of EIF3E-5a), which may be, for example, siRNA, shRNA, miRNA, antibodies and fragments thereof or other inhibitors specific for the FL form of EIF3E (exons 1-13, therefore in particular which have as target, in the case of RNA or antibodies, any target localised in exons 6-13).

Such a kit may be, in particular, a kit for the production of induced pluripotent stem cells (IPSCs) from human somatic cells or from somatic cells of other mammals.

For example, the kit may contain aliquots of mRNA for each gene of interest or directly mRNA cocktails which can be prepared and stored in "disposable" doses (doses sufficient for daily transfection) in order to avoid multiple freezing and thawing operations of the mRNAs, which could degrade.

Here also, an *in vitro* method is provided for the production of induced pluripotent stem cells (IPSCs) from somatic cells comprising the following steps:
a. introducing a protein as represented in SEQ ID NO 2 or a nucleic acid molecule encoding it and, concomitantly or subsequently, the human transcription factor SOX-2, the human Krueppel-like factor 4, the POU domain, class 5, transcription factor 1 and LIN28 or nucleic acids encoding them, in a human or in a non-human mammalian somatic cell, and
d. culturing said cell until it divides into a population of pluripotent cells optionally further comprising step b. before, concomitantly or subsequently to a. and before d
b. introducing the Myc proto-oncogene protein or a nucleic acid encoding it in said human or non-human mammalian somatic cell in a passage of cellular reprogramming and/or optionally further comprising step c. before, concomitantly or subsequently to a. and optionally c. and before d .:
c. introducing a down-regulator specific for EIF3E Full length which does not interfere with the regulation of the nucleic acid molecule as represented in SEQ ID NO 1 or 19, and/or of the protein as represented in SEQ ID NO 2 in a passage of cellular reprogramming, wherein said EIF3E Full length down-regulator is a nucleic acid selected from a siRNA, a shRNA a miRNA, an antibody specific for EIFE3E FL or fragments thereof that do not react with EIF3E-5a.

The description provides an *in vitro* method for the production of induced pluripotent stem cells (IPSCs) from somatic cells said method comprising the following steps:
a. introducing a nucleic acid that encodes for the protein having SEQ ID NO 2 and nucleic acids encoding, respectively, the human transcription factor SOX-2 proteins, the human transcription factor Krueppel-like 4 protein, the POU domain, class 5, transcription factor 1, and the protein LIN28 in a human or non-human mammalian somatic cell, and
d. culturing said cell until it divides into a population of pluripotent cells.
   The *in vitro* method according to the description will also comprise step b. before, subsequently to or concomitantly with a. and before d.:
b. introducing a nucleic acid that encodes the Myc proto-oncogene protein in said human or non-human mammalian somatic cell in a passage of cellular reprogramming.

In a particularly preferred embodiment said nucleic acids will be mRNA and the introduction will be performed by repeated transient transfection.

In addition, since a single strand of RNA is able to induce an immune response in the cells, N+B18R or other agents that inhibit the decrease of efficiency of transfection and cytotoxicity can be inserted in the culture medium.

The method as reported above improves the efficiency of cellular reprogramming compared with the method described in Takahashi K, Tanabe K, Ohnuki M, Narita M, Ichisaka T, Tomoda K, Yamanaka S. "Induction of pluripotent stem cells from adult human fibroblasts by defined factors". Cell. 2007 Nov 30;131(5):861-72 e in Takahashi K and Yamanaka S. Induction of Pluripotent Stem Cells from Mouse Embryonic and Adult Fibroblast Cultures by Defined Factors Cell 2006, 07 024.
The *in vitro* method according to the invention comprises step c. before, subsequently to or concomitantly with a. and optionally b. and before d.:
c. introducing a down-regulator specific for EIF3E full length and not for the protein having SEQ ID NO 2 as defined in the claims with said human or non-human mammalian somatic cell in a passage of cellular reprogramming.

The method comprising step c. also increases the efficiency of reprogramming compared with the method according to the invention devoid of step c. and is particularly indicated in order to increase the efficacy of reprogramming of senescent cells or cells taken from elderly individuals.

Alternatively, the method of the invention can be realised by inserting said sequences of nucleic acids into one or more lentiviral vectors and therefore inserting said vectors in the somatic cell of interest in accordance with standard methodologies.

The suitable nucleic acids, the suitable vectors, and the down-regulators of EIF3E FL are as described above.

In one embodiment, fibroblasts and somatic cells are transfected with mRNA encoding EiF3E-5a and EF3E-FL siRNA separately or together with the described reprogramming factors for the purpose of obtaining induced pluripotent cells or somatic stem cells that then can be re-differentiated as described in the method reported in detail and shown in Figure 8, which provides a summary.

In a particular embodiment, therefore, the method of the invention is a method in which said sequences of nucleic acids (as defined above) are in the form of RNA and said step of introduction in said cells is achieved by transient transfection with said nucleic acids.

The description also relates to human or non-human mammalian induced pluripotent stem cells (IPSC) obtainable with the methods described above.

Such cells can be used to produce differentiated somatic cells in accordance with the protocols known in the literature. Consequently, the invention also relates to an *in vitro* method for the production of induced pluripotent cells (iPS) or somatic stem cells comprising the steps of
a. producing human or non-human mammalian induced pluripotent stem cells by the method of the invention, and
b. subjecting the induced pluripotent stem cells thus obtained to a protocol of cellular differentiation.

The invention therefore relates also to the use of the protein having SEQ ID NO 2 or of a nucleotide molecule having SEQ ID NO 1 or 19 or of other nucleotide sequences which encode it for the reprogramming of human or non-human mammalian somatic cells in the methods described above and the use of the protein having SEQ ID NO 2 or of a molecule having the nucleotide sequence SEQ ID NO 1 or 19 (or of other nucleotide sequences that encode said protein) for the production of human or non-human mammalian induced pluripotent stem cells, still in the methods described above.

In addition, the description relates to the use of human or non-human mammalian induced pluripotent stem cells for the production *in vitro* of differentiated somatic cells; and to human or non-human mammalian induced pluripotent stem cells (IPSCs) obtainable by the methods according to the invention for use in therapies that require cellular reprogramming.

In another embodiment the description provides a therapeutic method that comprises the administration/inoculation of cells reprogrammed in accordance with the present description to patients in need of them.

Some of the pathologies that require the use of reprogrammed cells are, for example

Alzheimer's disease, rheumatoid arthritis, pulmonary fibrosis; acute myocardial infarction, liver cirrhosis, muscular dystrophy, optic atrophy and spinal muscular atrophy, autoimmune diseases; systemic erythematosus lupus: multiple sclerosis; autoimmune haemolytic anaemia; peripheral neuropathies: diabetes mellitus type 1 and 2, Basedow-Graves disease.

Lastly, the invention relates to an *in vitro* method for the production of induced pluripotent stem cells (IPSCs) from senescent somatic cells or from somatic cells taken from elderly individuals, comprising the use of a down-regulator specific for EIF3E Full Length which does not interfere with the regulation of molecules having SEQ ID NO 1 or 19 and/or SEQ ID NO 2 as defined in the claims.

In this case the method provides the addition of the down-regulator specific for EIF3E Full Length in any protocol already known for cellular reprogramming for the purpose of increasing the efficacy of reprogramming of senescent somatic cells or of somatic cells taken from elderly individuals.

The down-regulator specific for EIF3E Full Length is as defined above.

The methods and the agents according to the invention are suitable for the production of human or other mammalian iPSC cells, and the resultant cells are therefore human or other mammalian iPSC cells or human or other mammalian cells subsequently re-differentiated.

Particular advantages offered by the invention compared with other methods published for the generation of iPCS cells are based on the fact that the transient modulation of INT6-FL and of INT6-5a:
- makes it possible to increase the reprogramming efficiency and the generation of iPSC cells of terminally differentiated or senescent cells which demonstrate a low reprogramming efficiency on account of the fact that they are kept in a permanent state of closed chromatin
- does not comprise modifications of the transgenic type of the genome.
- reduces the risks of induction of a tumour.

The objective of the examples specified below is to show the scientific data obtained by the authors of the present invention and non-limiting ways for carrying out the invention.

### EXAMPLES

### I. Identification of the new gene INT6-5a.

The inventors describe the isolation of an isoform of the human transcript EIF3E Full-Length (INT6-FL), which has never been identified before. The new transcript was called INT6-5a (SEQ ID NO 1). The priority of the discovery of the new gene INT6-5a was recorded by the applicants of the invention at the National Institutes of Health of the United States of America (NIH-USA) with access number: GenBank: Banklt1589416 EIF3E KC333871 and will be available to the public from 31 December 2014. This isoform or any other short form of the human transcript EIF3E-FL have never been reported before now in the literature and therefore no function has ever before been attributed to the short human form of the transcript EIF3E-FL before now.

The human transcript EIF3E-5a, length 771 nt was generated by alternative splicing in respect of the transcript EIF3E full length. INT6-5a encodes for a protein that should be devoid of the nuclear localisation signal (NLS) and of the PCI (Proteasome COP9 Interacting) domain in accordance with the protein analyses carried out (Figures 1 and 2). Since the existence, expression, structure and function of the human gene EIF3E-5a has never been described before, the inventors have examined the level of expression and the role of the transcript of INT6-5a in human tissues, in primary cells and in cellular lines.

In mice the gene EIF3E encodes for the sub-unit P48 of the starting complex of the translation, complex EIF3 in eukaryotes, and has been identified as the site of integration (INTegration site 6, INT6) for the Mouse Mammary Tumour Virus (MMTV). The integration of the viral DNA in the mouse genome causes the formation of a cryptic stop sequence (stop codon), which generates the termination form of the transcript INT6-FL (Marchetti et al. 1995; Asano et al. 1997). Whereas the function of the termination form of the transcript INT6 of mice (mlnt6sh) has been described and noted, the present patent application is the first to describe the identification and the role of a short form of the human transcript INT6-FL.

The research conducted by the inventors also confirms that many functions of the human gene INT6-5a, described by other research groups, have been incorrectly attributed to the transcript INT6-FL due to siRNA technologies that use oligos which destroy both the INT6-FL form and the INT6-5a form at the same time.

### II. Expression profile of ITN6 FL and INT6-5a

The expression levels of both INT6-FL and INT6-5a were determined in normal and tumoral mammary tissues *in toto,* in tumorigenic and non-tumorigenic cell lines (TABLE I) and in primary cells isolated from normal and tumoral human mammary tissues. (Fluorescent Activated Cell Cytometry) with the antibodies EPCAM and CD49f (Figure 3). The comparison of the expression levels of INT6-FL and INT6-5a in normal and tumoral mammary tissues *in toto* (patients 21-31) and in the lines MCF7 and MCF10A shows that these are expressed at significantly low levels (1000 and 1660 copies per cell respectively) in the INT6-FL cell lines compared with tumoral tissues (on average 4,000 copies per cell), but above all compared with healthy tissues (on average 15,000 copies per cell) (see TABLE I above).

Since the MCF7 cells are tumorigenic and the MCF10A cells are non-tumorigenic, but both demonstrate very low levels of INT6-FL compared with the tissues *in toto,* the authors of the present invention have hypothesised that the strong down-regulation of INT6-FL is not directly associated with the tumorigenic capacity of the line, but with the process of immortalisation of the cells, and that the high levels of INT6-FL in the cells derived from healthy tissue could be associated with their degree of differentiation and the low level of proliferation at which the cells absolutely must be kept in adult tissue.

In order to investigate this hypothesis, primary cells were isolated from normal mammary tissue and were propagated under culture conditions with different concentrations of serum (10 % and 15 %) in order to modulate the cell proliferation (see cells of patients Br15, Br5 and Br38 cultivated in 10 % and 15 % serum), or were propagated in culture in the long term in order to induce senescence (see Br83 with late passages).

The primary cells kept in culture with reduced concentrations of serum (10 %) and primary cells cultivated in the long term both demonstrated a reduction of the cell proliferation and significantly high expression levels of INT6-5a. The primary cells kept in culture in the long term enter a permanent state of proliferative interruption associated with the onset of senescence and are characterised by an extreme insensitivity to mitotic stimuli and by a condition of repressive chromatin, which induces the conversion of the euchromatin into heterochromatin and prevents the transcription of genes that promote growth. The primary cells propagated in conditions of low serum also enter into a state of heterochromatin caused instead by the temporary state of proliferative dormancy caused by the culture conditions, which slow down proliferation.

The results suggest that the high expression of INT6-FL induces or is associated with the state of heterochromatin that may be caused by the temporary proliferative dormancy induced by the culture conditions with low serum or by the onset of senescence in cells propagated in culture in the long term.

In order to confirm whether the high expression of INT6-FL is associated with the temporary state of proliferative dormancy linked to culture conditions that block proliferation or to a permanent state of proliferative interruption associated with the terminal phase of the differentiation or with the onset of senescence, the expression level of INT6-FL was assessed in populations of cells enriched by terminally differentiated and/or senescent luminal cells and in basal-like/stem/progenitor cells sorted by cytofluorometry (Fluorescent Activated Cell Cytometry Sorter, FACS) with EPCAM (markers for differentiated luminal cells) and CD49f (markers of undifferentiated basal-like cells) antibodies on the basis of cells isolated from normal human dissociated mammary tissue (Figure 3 top of box).

It has been observed that the population P4 (EPCAM^{high} and CD49f^{low}) which shows the most differentiated cells indicates significantly high levels of INT6-FL, both compared with MCF7 and MCF10 (MCF10A not shown in this figure) and compared with the population PF that contains undifferentiated basal-like/stem/progenitor mammary cells (EPCAM^{low} and CD49f^{high}), confirming that the high expression of INT6-FL is associated with the terminally differentiated and/or senescent cells (Figure 3, bottom of box).

It has also been observed that the basal-like/stem/progenitor mammary cells, although they have lower levels of INT6-FL compared with mature luminal cells, demonstrate the highest levels of INT6-5a ever seen (Figure 3).

In order to further confirm this hypothesis, the expression levels of INT6-FL in infantile fibroblasts and in mammary cells propagated so as to become senescent were determined and were compared with the levels of INT6-FL in immortalised MCF7 and MCF10 cell lines (Figure 4, only mammary fibroblasts and mammary luminal cells are shown). The senescent cells do not have any ability to remodel the chromatin, nor to modify the gene expression or the proliferation insofar as they are kept in a permanently closed state of the chromatin (heterochromatin state). The senescent cells demonstrate high levels of INT6-FL compared with the other culture conditions or the MCF7 or MCF10A cell lines, suggesting that the up-regulation of INT6-FL is associated with a conformation of compact heterochromatin.

On the basis of preliminary data, which cannot be confirmed, the authors of the present invention have observed that senescent mammary fibroblasts and normal mammary cells have at least 150 times more INT6-FL compared with non-senescent primary cell lines.

Collectively, the results obtained suggest that when the normal primary cells reach the state of senescence, INT6-FL is significantly up-regulated compared with cell lines (MCF7 or MCF10A, Figure 4) or normal primary cells in active division (see cells cultivated in 15 % serum) or compared with primary cells isolated from tumors also in active proliferation (tumoral samples in Table 1), and that the loss or the significant down-regulation of INT6-FL confirms in normal and tumoral cells that these have acquired a state of immortalisation (MCF7 and MCF10A) and is associated with an open conformation of the chromatin.

The authors of the present invention have therefore concluded that the high levels of expression of INT6-FL in normal mammary tissue compared with tumoral tissue (Table 1, patients 21-32) are caused by a greater number of terminally differentiated or senescent luminal cells present in the normal tissue compared with the tumoral tissue. This observation may also suggest that, with the development of the tumour, the luminal stem/progenitor cells cannot enter into the terminal differentiative state and/or that the terminally differentiated or senescent luminal cells become de-differentiated and gain the ability to proliferate indefinitely (or the capability of immortalisation) accompanied by down-regulation of INT6-FL.

In addition, since the tumoral and non-tumoral cell lines (MCF7 and MCF10A) both have a very low expression of INT6-FL compared with mammary tissue *in toto,* it is possible to conclude that the loss or down-regulation of INT6-FL is associated with the immortalisation of any type of cell, irrespective of whether tumorigenic or non-tumorigenic.

### III. The gene INT6-5a controls the state of the chromatin and the translation of the RNA of the histones.

It has recently been demonstrated that INT6-FL is necessary for the effective translation of the RNA of the histones in human cells (Neusiedler et al. 2012). The authors of the present invention have assumed that the absence of the functional domains NLS and PCI could induce INT6-5a to act as a dominant-negative transcript, which in normal or tumoral cells regulates the translation of the RNA of the histones independently of INT6-FL.

It has been observed that mature luminal cells (population P4, EPCAM^{high} and CD49f^{low} in Figure 3) have a significantly higher content of histones compared with basal-like/stem/progenitor mammary cells (population P6, EPCAM^{low} and CD49f^{high}) or all MCF7 cells.

The up-regulation of INT6-5a induces the down-regulation of the protein of the histone H3 in normal and tumoral mammary cells (Figure 5, reported for MCF10A). Since the up-regulation of INT6-5a has not influenced the expression of the protein INT6-FL (Figure 5, panel at the bottom), it is possible to assert that the activity of INT6-5a is independent of the activity of INT6- FL.

This observation suggests that, since the terminally differentiated or senescent cells have a low index of proliferation, they present a closed conformation of the chromatin and have a different pattern of proteins associated with the chromatin, but also have high levels of expression of INT6-FL, and INT6-FL could be associated with the heterochromatin state of the differentiated and/or senescent cells. Similarly, since the basal-like/stem/progenitor mammary cells (population P4, EPCAM^{low} CD49f^{high}) are in an open chromatin conformation, they have lower levels of INT6-FL compared with mature luminal cells, but have higher levels of INT6-5a, and INT6-5a could be associated with the open chromatin state of undifferentiated stem cells.

### Materials and methods

### 1. Cell lines

MCF7 cells (a human epithelial tumorigenic mammary cell line ATCC® HTB-22 ™) were cultivated in medium RPMI-1640 (Invitrogen, GIBCO Laboratories, cat. N. 21.875.091) supplemented with 10 % foetal bovine serum (Sigma, cat. N. F7525), 10 µg/insulin ml (Sigma, cat. N. I9278) and penicillin-streptomycin (Invitrogen, GIBCO Laboratories, cat. N. 15.140.122). The cells were sewn in a density of 5000 cells/cm2 and cultivated in 5 % CO2 at 37 °C.

MCF10A cells (a human epithelial non-tumorigenic mammary cell line ATCC® CRL-10317 ™) were cultivated in DMEM-Glutamax/F-12 (Invitrogen GIBCO Laboratories, cat. N. 31.966.047 e N. 11.765-054) supplemented with 5 % equine serum inactivated under heat (Invitrogen, GIBCO Laboratories, cat. N. 16050130), 50 µM of cyclic AMP butyryl (Sigma, cat. N. D0627), 10 µg/ml of insulin (Sigma, cat. N. I9278), 20 ng/ml of recombinant mouse EGF (Sigma, cat. N. E4127), 500 ng/ml of hydrocortisone (Sigma, cat. N. H0888) and penicillin-streptomycin (Invitrogen, GIBCO Laboratories, cat. N. 15.140.122). The cells were sewn in a density of 25000 cells/cm2 and cultivated in 5 % CO2 at 37 °C.

### 2. Dissociation of the tissue and preparation of primary cells

The mammary tissue was obtained from informed patients by mammary reduction and was collected and mechanically and enzymatically dissociated. The mammary tissue was cut into small pieces using surgical scissors and was incubated for 4 hours at 37 °C, 5 % CO2 in DMEM-KO (Invitrogen GIBCO Laboratories, cat. N. 10.829.018) 10 % KnockOut™ serum (Invitrogen GIBCO Laboratories, cat. N. 10828-028) supplemented with 300 U/ml collagenase (Sigma, cat. N. C0130) and 100 U/ml hyaluronidases (Sigma, cat. N. H3506). After the enzymatic treatment, the tissues were collected in a falcon tube, centrifuged at 0.2xg for 10 minutes, washed once with DMEM-KO and filtered using filters for cells from 40 µicron (Fisher Scientific). Single cells were harvested and cultivated in DMEM-Glutamax/F-12 (Invitrogen GIBCO Laboratories, cat. N. 31.966.047 and 11.765-054) 10 % KnockOut™ serum (Invitrogen, GIBCO Laboratories, cat. N.10.828-028) 10 ng/microlitre EGF (Sigma, cat. N. E4127), 1 µg/ ml insulin (Sigma, cat. N. I9278) 0.5 µg/ml hydrocortisone (Sigma, cat. N. H0888), 4 µg/ml bFGF (Sigma, cat. N. F0291). The cells were placed on plates in a density of 4000 cells/cm² and divided every 3-4 days by trypsinisation (Invitrogen, GIBCO Laboratories, cat. N. 25.200.072). The culture plates were coated with 0.1 % gelatin (Sigma, cat. N. G1393) to improve the cellular adhesion.

### 3. Preparation of senescent cultures.

The primary cells obtained as indicated above (Br 15, Br 5, Br83 and Br95) were cultivated in medium DMEM-Glutamax/F-12 (Invitrogen GIBCO Laboratories, cat. N. 31.966.047 e 11.765-054) and 15 % serum (Sigma, cat. N. F7525) for 8 or 12 weeks with continuous passages until reaching senescence.

### 4. Protocol of transfection of the siRNA oligos for the down-regulation of EIF3E-FL into MCF10A cells

MCF10A cells (ATCC) were placed on plates the day before transfection in a density of 22000 cells per cm² on 12-well plates, in MEGM complete commercial medium (Lonza).
Before the treatment, siRNA medium was replaced with 1 ml fresh medium
The siRNA oligomers used are listed below
1) for inhibition of the expression levels of the gene EIF3E (NM_001568), two double-strand siRNA oligonucleotides were used (Sigma): SASI_Hs01_00166672 (5'-GUUUAUAUGUUAACUUUGA-3') and SASI_Hs01_00166675 (5'-CAGUGUAUCAGCAUUAACA-3'). These siRNA oligonucleotides were used together in a concentration of 25 nM each;
An siRNA was used as negative control: MISSION® siRNA universal negative control # 2 (Sigma), both in a concentration of 50 and in a concentration of 100 nM. The siRNAs were diluted in 100 microlitres of OptiMEM (Invitrogen, GIBCO Laboratories) and at the same time 4 microlitres of Interferin (PolyPlus transfection) were diluted in 100 microlitres of OptiMEM. The two solutions were mixed and incubated for 10 minutes at ambient temperature. Then, the 200 microlitres of siRNA and Interferin were added to the wells. The cells were harvested after 8, 24 and 48 hours.

### 5. Preparation of lentiviral constructs and translation of the cells

The sequence INT6-5a was cloned in the vector pCDH-CMV-MCS-EF1-copGFP (System Biosciences SBI, cat. N. CD511B-1) after having been obtained by amplification of cDNA converted from mRNA isolated from MCF10A cells using primers having SEQ ID 15 and 16.

The precision of the sequence of INT6-5a was confirmed by sequencing. The lentiviral particles were produced in HEK-293T cells, transfecting pCDH-CMV-MCS-EF1-copGFP or pCDH-CMV-INT6-5a-EF1-copGFP with the support vectors psPAX2 and pMD2.G (Sistema Biosciences, SBI. Cat. N. CD511B-1) in a ratio of 3: 2: 1 in the presence of Lipofectamine™ 2000 Transfection Reagent (Life Technologies, cat. N. 11.668.019). The lentiviral particles were concentrated with the solution PEG-ITTM (Virus Precipitation Solution, System Biosciences, SBI, cat. N. LV810A-1 /). The cells were placed on plates in adhering conditions and after 24 hours the cells were infected by adding the viral preparation directly to the culture medium, with the addition of 8 micrograms/ml of hexadimethrine bromide (Sigma, cat. N. H9268). In order to prevent toxicity, the culture medium was substituted after 24 hours. The cells were then translated with lentiviruses expressing INT6-5a and GFP, and cells infected with lentiviruses expressing only GFP were used as control.

### 6. Quantitative RT-PCR

The RNAs were extracted from MCF7, MCF10A and from primary human cells using the reagent TriZol™, in accordance with the manufacturer's instructions (Life Technology, cat. N. 15.596.018). The cDNAs were synthesised using 1 □microg of total RNA and the Master Mix High capacity RNA-to-cDNA (Applied Biosystems, cat. N. 4.368.813).

Sequences of the sense and antisense primers used for the quantitative RealTime reactions in SybrGreen (Applied Biosystems, cat. N. 4.309.155.)
SEQ ID NO 3
   EIF3E-5a sense: 5'-GCATGGTTTTAGGCAGGAATATTT-3'
SEQ ID NO 4
   EIF3E-5a antisense: 5'-CTTCAGGCCAGGAGTTTGAGA-3'
SEQ ID NO 5
   EIF3E sense (NM_001568): 5'- CAGATGTTGGCCATGAATATTGA-3'
SEQ ID NO 6
   EIF3E antisense (NM_001568): 5'-GCCCAGTTAGGAGCCTCTGA-3'
SEQ ID NO 7
   GAPDH (NM_002046) sense: 5'-GCACCGTCAAGGCTGAGAAC-3'
SEQ ID NO 8
   GAPDH (NM_002046) antisense: 5'- AGGGATCTCGCTCCTGGAA-3'
SEQ ID NO 9
   HPRT1 (NM_000194) sense: 5'-TTTGCTGACCTGCTGGATTACA-3'
SEQ ID NO 10
   HPRT1 (NM_000194) antisense: 5'-GGTCATTACAATAGCTCTTCAGTCTGAT-3'
SEQ ID NO 11
   Rpl32 sense (NM_000994): 5'-TTCATCCGGCACCAGTCA-3'
SEQ ID NO 12
   Rpl32 antisense (NM_000994): 5'-GTCAATGCCTCTGGGTTTCC-3'
SEQ ID NO 13
   Rpl34 sense (NM_000995): 5'-TTTGACATACCGACGTAGGCTTT-3'
SEQ ID NO 14
   Rpl34 antisense (NM_000995): 5'-GGGTTCGGGACAGCCTAGTT-3'
SEQ ID NO 17
   SASI_Hs01_00166672 5'-GUUUAUAUGUUAACUUUGA-3'
SEQ ID NO18
   SASI_Hs01_00166675 5'- CAGUGUAUCAGCAUUAACA-3'.

The RealTime PCR was carried out using the Applied Biosystems 7500 apparatus.

### 7. Generation of the iPSC cells by conventional method with lentivirus

The protocol used for the generation of human IPS cells is substantially very similar to that presented in the following publications

Takahashi K, Tanabe K, Ohnuki M, Narita M, Ichisaka T, Tomoda K, Yamanaka S. Induction of Pluripotent Stem Cells from Adult Human Fibroblasts by Defined Factors Cell, 2007, 11: 19

Takahashi K and Yamanaka S. Induction of Pluripotent Stem Cells from Mouse Embryonic and Adult Fibroblast Cultures by Defined Factors Cell 2006, 07024.

In order to execute the protocol, the constructs prepared from points 4 and 5 were used
and the following approach was adopted:
1- Translation of embryonic mouse fibroblasts (MEF) with lentiviruses FUW-TetO-OSKM (cod. n. 20321 inducible, polycistronic, expressing the "4-factors") and transactivator thereof, (FUW-M2rtTA cod. n. 20342)
2- Test to assess the % of translation by immuno for Oct4 and RealTime for the integrations (results >70%)
3- Amplification of the cells
4- Translation of the lentivirus expressing EIF3E-5a prepared as per point 5.
5- The day following medium exchange and start of induction with doxicycline (day 1 of the reprogramming)
6- Exchange of half medium every 2 days with fresh doxycycline and LIF.
7- On day 25 the formed colonies are individually pricked, subjected to single-cell trypsinisation, placed on a new plate on a carpet of fibroblasts and cultivated with medium specific for mES with LIF but without doxycycline. (NOTE: the removal of the doxycycline includes the extinguishing of the reprogramming factors).
8- The colonies reformed in 3-5 days and had acquired and maintained the typical morphology of the colonies of the embryonic stem cells (see Figure 7), expressing the protein GFP, a reporter that reveals the re-expression of the endogenic Oct4 and indicates the reprogramming occurred. The efficiency of the reprogramming in the condition with EIF3E-5a was doubled compared with the condition with the "4 factors" only.

### 8. Generation of the iPSCs cells by way of repeated transient transfection

100,000, 50,000 and 25,000 human fibroblasts were sewn in a 6-well plate into different wells and were cultivated at 37 °C and 5 % CO2, in DMEM (Dulbecco Modified Eagle Medium) and in 10 % foetal bovine serum to achieve a confluence varying between 50 and 90 % in 24 or 48 hours. The different concentrations of fibroblasts were used, since cells derived from patients demonstrate variability in the reprogramming and in the cell proliferation. Three hours before transfection, the medium was removed and 2 ml of fresh medium were added.

The generation of iPSCs was performed with success using different combinations of genes compared with the original protocol (Yamanka et al.) and comprised cocktails of genes including mRNAs of the isoform EIF3E-5a and siRNA against the isoform IEF3E FL and a cocktail that included mRNA of Oct-4, Sox2, Klf4, cMyc and LIN28.

The weight/volume ratio of the reagents in the transfection reaction was maintained between 2 and 6 µl per microgram of RNA. Since the efficiency of transfection of mRNA is dependent on the purity and on the structure of the mRNA, the amounts of EIF3E-FL and/or EIF3E-5a must be optimised for each type of cell. In addition, since a single strand of RNA is able to induce an immune response in the cells, B18R was used in the medium to inhibit the reduction of efficiency of transfection and cytotoxicity (Alcami et al, 2000, Journal of Virology 74 (230: 11.230-11.239).

### Preparation of the reagents

### A. Preparation of the cocktail for the generation of iPSCs

Step 1: Remodelling of the chromatin to allow a permissive chromatin state. Two transfections repeated every 24 hours from day 0 to day 2, using
   60 pmol of siRNA per EIF3E-FL, or
   1 microgram of mRNA per EIF3E-5a or
   60 pmol of siRNA per EIF3E-FL + 1 microgram of mRNA per EIF3E-5a, depending on the cell type.
Step 2: Reprogramming. Transfections repeated every 24 hours from day 2 to day 20, using a master mix containing Oct-4 (Stemgent cat n. 05-0014) Sox2 (Stemgent cat n. 05-0015), Klf4 (Stemgent cat n. 05-0016), cMyc (Stemgent cat n. 05-0016), LIN28 (Stemgent cat n. 05-0017), nGFP used as reporter (Stemgent cat n. 05-0019) in molar stoichiometry of 3: 1: 1: 1: 1: 1 respectively.

### B. Preparation of cocktail for the generation of somatic stem cells iSSCs or of differentiated cells iDSs

Step 1. Remodelling of the chromatin. Two transfections repeated every 24 hours from day 0 to day 2, using
   60 pmol of siRNA per EIF3E-FL, or
   1 microgram of mRNA per EIF3E-5a or
   60 pmol of siRNA per EIF3E-FL + 1 microgram of mRNA per EIF3E-5a, depending on cell type.
Step 2. Reprogramming. Perform transfections repeated every 24 hours from day 2 to day 20, using a master mix which must be determined with factors specific for the somatic stem cell that is to be obtained or with factors that allow the specific differentiation of the cells to be obtained.
The mRNA cocktails were prepared and stored in "disposable" doses for the number of wells to be transfected every day in order to avoid multiple freezing and thawing operations of the mRNAs, which could degrade.

At the same time, the lentiviral vectors already used in the method described by Yamanaka (above) acquired from Addgene (Plasmid repository) obtained in accordance with the protocol described in Carey et al. 2008 2009 were also used as proof of principle in addition to the lentiviral vector prepared under point 5. (B.W., Markoulaki S., Hanna J., Saha K., Gao O., Mitalipova M., Rudolf Jaenisch R. Reprogramming of murine and human somatic cells using a single polycistronic vector PNAS_2009_vol. 106_no. 1_157-162)
Examples of codes of the constructs and the links to the constructs available from Addgene:
cod.20321 - FUW-TetO-OSKM (inducible): https://www.addgene.org/20321/
cod.20342 - FUW-M2rtTA (transactivator): https://www.addgene.org/20342/
cod. 20325 - FUW-SOKM (constitutive): https://www.addgene.org/20325/
A comparative test was also performed, of which the results are shown in Figure 7, in which there are compared the reprogramming protocol followed by the traditional cocktail containing the "4-factors" from the literature, without EIF3E-5a (box on the right) and with EIF33-5a (box on the left). The data obtained shows that with the addition of the mRNA of EIF3E-5a, the efficiency of formation of colonies appears to be significantly increases (doubled).

### BIBLIOGRAPHY

Alcamí A, Symons JA, Smith GL.The vaccinia virus soluble alpha/beta interferon (IFN) receptor binds to the cell surface and protects cells from the antiviral effects of IFN. J Virol. 2000 74:11230-11239.
Asano K, Merrick W C, Hershey J W, et al. The translation initiation factoeIF3-p48 subunit is encoded by int-6, a site of frequent integration by the mouse mammary tumovirus genome J BiolChem, 1997, 272, 23477
Carey B.W., Markoulaki S., Hanna J., Saha K., Gao O., Mitalipova M., Rudolf Jaenisch R. Reprogramming of murine and human somatic cells using a single polycistronic vector PNAS_2009_vol. 106_no. 1_157-162
Eilers M, Eisenman RN. Myc's broad reach. Genes Dev. 2008, 22, 2755
Hayflick L, Moorhead PS. 1961. The serial cultivation of human diploid cell strains. Exp Cell Res 25: 585-621.
Lapasset L, Milhavet O, Prieur A, et al. Rejuvenating senescent and centenarian human cells by reprogramming through the pluripotent state Genes Dev. 2011 25, 2248
Lister R, Pelizzola M, Kida YS, David Hawkins D, Nery JR, Hon G, Antosiewicz-Bourget J, O'Malley R, Castanon R, Klugman S, Downes M, Yu R, Stewart R, Ren B, Thomson JA, EvansRM, & Ecker JR Hotspots of aberrant epigenomic reprogramming in human induced pluripotent stem cells Nature, 2011,471
Marchetti A, Buttita F, Miyazaki S, Gallahan D, Smith G, Callahan R, Int-6, a highly conserved,widely expressed gene, is mutated by mouse mammary tumovirus in mammary preneoplasia. J Virol, Vol. 69, 1995,1932.
Marchetti A, Buttitta F, Pellegrini S, Bertacca G, Callahan R. Reduced expression of INT-6/eIF3-p48 in human tumors. Int J Oncol, 2001, 18,175.
Neusiedler J, Mocquet V, Limousin T, Ohlmann T, Morris C, Jalinot P. INT6 interacts with MIF4GD/SLIP1 and is necessary foefficient histone mRNA translation. RNA, 2012, 18, 1163.
Polo JM, Anderssen E, Walsh RM, Schwarz BA, Nefzger CM, Lim SM, Borkent M, Apostolou E, Alaei S, Cloutier J, Bar-Nur O, Cheloufi S, Stadtfeld M, Figueroa ME, Robinton D, Natesan S, Melnick A, Zhu J, Ramaswamy S, Hochedlinger K. A Molecular Roadmap of Reprogramming Somatic Cells into iPS Cells Cell 2012.11.039
Takahashi K, Tanabe K, Ohnuki M, Narita M, Ichisaka T, Tomoda K, Yamanaka S. Induction of Pluripotent Stem Cells from Adult Human Fibroblasts by Defined Factors Cell. 2007 Nov 30;131(5):861-72.
Takahashi K and Yamanaka S. Induction of Pluripotent Stem Cells from Mouse Embryonic and Adult Fibroblast Cultures by Defined Factors Cell 2006, 07 024
Thomas Kuilman, Chrysiis Michaloglou, Wolter J. Mooi, et al. The essence of senescence Genes Dev. 2010, 24: 2463
Iwan Walev, Sebastian Chakrit Bhakdi, Fred Hofmann, Nabil Djonder, Angela Valeva, Klaus Aktories and Sucharit Bhakdi "Delivery of proteins into living cells by reversible membrane permeabilization with streptolysin-O" PNAS March 13, 2001 vol. 98 no. 6 3185-3190
Warren L, Manos PD, Ahfeldt T, Loh YH, Li H, Lau F, Ebina W, Mandal PK, Smith ZD, Meissner A, Daley GQ, Brack AS, Collins JJ, Cowan C, Schlaeger TM, Rossi DJ. Highly efficient reprogramming to pluripotency and directed differentiation of human cells with synthetic modified mRNA. Cell Stem Cell. 2010 7:618-30
Claire O. Weill, Stéphanie Biri, Abdennaji Adib, and Patrick Erbacher "A practical approach for intracellular protein delivery" Cytotechnology. Jan 2008; 56(1): 41-48. Published online Oct 16, 2007. doi: 10.1007/s10616-007-9102-3. Yu et al, "Induced pluripotent stem cell lines derived from human somatic cells" Science. 2007 Dec 21;318(5858):1917-20. Epub 2007 Nov 20

## Claims

1. A nucleic acid molecule as represented in SEQ ID NO 1 or 19 encoding an isoform of the human protein EIF3E that does not comprise exons 6-13.

2. A protein as represented in SEQ ID NO 2 wherein said protein is an isoform of the human protein EIF3E (EIF3E-5a).

3. An agent for the production of IPSCs cells from somatic human or non-human mammalian cells comprising the protein as represented in SEQ ID NO 2 or a nucleic acid molecule as represented in SEQ ID NO 1 or 19, or a nucleic acid molecule encoding the protein of SEQ ID NO 2.

4. The agent according to claim 3 further comprising a down-regulator specific for EIF3E Full Length (EIF3E FL) which does not interfere with the regulation of SEQ ID NO 1 or 19, and/or of SEQ ID NO 2, optionally further comprising the human transcription factor SOX-2, the human Krueppel-like factor 4, the POU domain, class 5, transcription factor 1 and LIN28 or nucleic acids that encode them, wherein said EIF3E FL down-regulator is a nucleic acid selected from a siRNA, a shRNA a miRNA, an antibody specific for EIF3E FL or fragments thereof that do not react with EIF3E-5a.

5. The agent according to claim 4 further comprising the Myc proto-oncogene protein or a nucleic acid encoding it.

6. The agent according to any one of claims 4 tor 5 wherein said nucleic acids are inserted, each, in one or more lentiviral vector or wherein said nucleic acid molecules are mRNAs.

7. An *in vitro* method for the production of induced pluripotent stem cells (iPSC) from somatic cells comprising the following steps:
a. introducing a protein as represented in SEQ ID NO 2 or a nucleic acid molecule encoding it and, concomitantly or subsequently, the human transcription factor SOX-2, the human Krueppel-like factor 4, the POU domain, class 5, transcription factor 1 and LIN28 or nucleic acids encoding them, in a human or in a non-human mammalian somatic cell, and
d. culturing said cell until it divides into a population of pluripotent cells optionally further comprising step b. before, concomitantly or subsequently to a. and before d .:
b. introducing the Myc proto-oncogene protein or a nucleic acid encoding it in said human or non-human mammalian somatic cell in a passage of cellular reprogramming and/or optionally further comprising step c. before, concomitantly or subsequently to a. and optionally c. before d .:
c. introducing a down-regulator specific for EIF3E FL which does not interfere with the regulation of the nucleic acid molecule as represented in SEQ ID NO 1 or 19, and/or of the protein as represented in SEQ ID NO 2 in a passage of cellular reprogramming, wherein said EIF3E FL down-regulator is a nucleic acid selected from a siRNA, a shRNA a miRNA, an antibody specific for EIF3E FL or fragments thereof that do not react with EIF3E-5a.

8. The method according to claim 7, wherein said somatic human or non-human mammalian cell is a senescent cell or is from elderly individuals.

9. The method according to any one of claims 7 or 8 wherein said nucleic acid molecules are respectively inserted in one or more lentiviral vector or wherein said nucleic acid molecules are in the form of mRNA and said step of introduction in said cell is achieved by repeated transient transfections with said nucleic acid molecules.

10. The method according to claim 8, wherein a down-regulator specific for EIF3E FL which does not interfere with the regulation of nucleic acid molecules as represented in SEQ ID NO 1 or 19, and/or of the protein as represented in SEQ ID NO 2 as defined in claim 7 is further introduced in said cell in a passage of transfection concomitant or subsequent to the introduction of said mRNA encoding a protein as represented in SEQ ID NO 2 and before the transfection steps of mRNAs encoding the human transcription factor SOX-2, the human Krueppel-like factor 4, the POU domain, class 5, transcription factor 1, LIN28 and optionally the Myc proto-oncogene protein.

11. The method according to claim 10 wherein said human or non-human mammalian somatic cell is a senescent cell or is from elderly individuals.

12. An in vitro method for optimizing the production of induced pluripotent stem cells (iPSC) from a senescent somatic cell or from a somatic cell from elderly individuals comprising introducing into said cell a protein as represented in SEQ ID NO 2 or a nucleic acid molecule encoding it, and a down-regulator specific for EIF3E FL which does not interfere with the regulation of nucleic acid molecules as represented in SEQ ID NO 1 or 19, and/or of the protein as represented in SEQ ID NO 2, before and during the steps of cellular reprogramming, wherein said EIF3E FL down-regulator is a nucleic acid selected from a siRNA, a shRNA a miRNA, an antibody specific for EIF3E FL or fragments thereof that do not react with EIF3E-5a.

13. An *in vitro* method for the production of differentiated somatic cells comprising the steps of
a. producing human or non-human mammalian induced pluripotent stem cells by carrying out the method according to any one of claims 7 to 11, and
b. submitting the induced pluripotent stem cells thus obtained to a protocol of cell differentiation.

14. Use of the protein as represented in SEQ ID NO 2 or of the nucleic acid molecules as represented in SEQ ID NO 1 or 19, or of other molecules with nucleotide sequences encoding said protein as represented in SEQ ID NO 2 for the in vitro production of human or non-human mammalian induced pluripotent stem cells.

15. A kit for preparing human or non-human mammalian induced pluripotent stem cells comprising one or more aliquots of an agent as defined in claims 3 to 7.

## Patentansprüche

1. Nukleinsäuremolekül gemäß SEQ ID NO 1 oder 19, das eine Isoform des Humanproteins EIF3E kodiert, das keine Exons 6-13 umfasst.

2. Protein gemäß SEQ ID NO 2, wobei das Protein eine Isoform des Humanproteins EIF3E (EIF3E-5a) ist.

3. Agens zur Herstellung von IPSCs-Zellen aus somatischen humanen oder nichthumanen Säugetierzellen, das umfasst: das Protein gemäß SEQ ID NO 2 oder ein Nukleinsäuremolekül gemäß SEQ ID NO 1 oder 19, oder ein Nukleinsäuremolekül, das das Protein von SEQ ID NO 2 kodiert.

4. Agens gemäß Anspruch 3, das weiterhin umfasst: einen Herabregulierer für Vollängen-EIF3E (EIF3E FL), der die Regulierung von SEQ ID NO 1 oder 19, und/oder von SEQ ID NO 2, nicht stört, optional weiterhin umfasst: den humanen Transkriptionsfaktor SOX-2, den humanen Krüppel-Like-Faktor 4, den POU-Domäne (Klasse 5)-Transkriptionsfaktor 1 und LIN28 oder Nukleinsäuren, die sie kodieren, wobei der EIF3E FL-Herabregulierer eine Nukleinsäure ist, die aus der folgenden Liste ausgewählt wird: eine siRNA, eine shRNA, eine miRNA, ein spezifischer Antikörper für EIF3E FL oder Fragmenten davon, der nicht mit EIF3E-5a reagiert.

5. Agens gemäß Anspruch 4, das weiterhin umfasst: das Myc-Protoonkogenprotein oder eine Nukleinsäure, die es kodiert.

6. Agens gemäß einem der Ansprüche 4 bis 5, wobei die Nukleinsäuren jeweils in einen oder mehrere lentivirale Vektoren eingefügt sind, oder wobei die Nukleinsäuremoleküle mRNAs sind.

7. In-vitro-Verfahren zur Herstellung von induzierten pluripotenten Stammzellen (iPSC) aus somatischen Zellen, das die folgenden Schritte umfasst:
a. Einführen eines Proteins gemäß SEQ ID NO 2 oder eines Nukleinsäuremoleküls, das es kodiert, und gleichzeitig oder nachfolgend des humanen Transkriptionsfaktors SOX-2, des humanen Krüppel-Like-Faktors 4, des POU-Domäne (Klasse 5)-Transkriptionsfaktors 1 und LIN28 oder Nukleinsäuren, die sie kodieren, in eine humane oder in eine nicht-humane somatische Säugetierzelle, und
d. Kultivieren der Zelle, bis sie sich in eine Population von pluripotenten Zellen teilt, optional weiterhin umfassend: Schritt b) vor, gleichzeitig mit oder nachfolgend auf a) und vor d):
b. Einführen des Myc-Protoonkogenproteins oder einer Nukleinsäure, die es kodiert, in humane oder nicht-humane somatische Säugetierzellen bei einem Durchgang einer zellulären Neuprogrammierung und/oder optional weiterhin Umfassen von Schritt c) vor, gleichzeitig mit oder nachfolgend auf a) und optional c) vor d):
c. Einführen eines für EIF3E FL spezifischen Herabregulierers, der die Regulation des Nukleinsäuremoleküls gemäß SEQ ID NO 1 oder 19 und/oder des Proteins SEQ ID NO 2 nicht stört, in einen Durchgang einer zellulären Neuprogrammierung, wobei der EIF3E FL-Herabregulierer eine Nukleinsäure ist, die aus der folgenden Gruppe ausgewählt wird: eine siRNA, eine shRNA, eine miRNA, ein spezifischer Antikörper für EIF3E FL oder Fragmenten davon, der nicht mit EIF3E-5a reagiert.

8. Verfahren gemäß Anspruch 7, wobei die somatische humane oder nicht-humane Säugetierzelle eine seneszente Zelle ist oder von älteren Probanden stammt.

9. Verfahren gemäß einem der Ansprüche 7 oder 8, wobei die Nukleinsäuremoleküle jeweils in einen oder mehrere lentivirale Vektoren eingeführt werden, oder wobei die Nukleinsäuremoleküle in der Form von mRNA vorliegen und der Schritt zur Einführung in die Zelle durch wiederholte transiente Transfektionen mit den Nukleinsäuremolekülen erreicht wird.

10. Verfahren gemäß Anspruch 8, wobei ein für EIF3E FL spezifischer Herabregulierer, der die Regulation von Nukleinsäuremolekülen gemäß SEQ ID NO 1 oder 19, und/oder des Proteins gemäß SEQ ID NO 2 gemäß Anspruch 7 nicht stört, weiterhin in die Zelle bei einer Passage einer Transfektion eingeführt wird, gleichzeitig mit oder nachfolgend auf die Einführung der mRNA, die ein Protein gemäß SEQ ID NO 2 kodiert, und vor den Transfektionsschritten von mRNAs, die den humanen Transkriptionsfaktor SOX-2, den humanen Krüppel-Like-Faktor 4, den POU-Domäne (Klasse 5)-Transkriptionsfaktor 1, LIN28 und optional das Myc-Protoonkogenprotein kodieren.

11. Verfahren gemäß Anspruch 10, wobei die humane oder nicht-humane somatische Säugetierzelle eine seneszente Zelle ist oder von älteren Probanden stammt.

12. In-vitro-Verfahren zum Optimieren der Herstellung von induzierten pluripotenten Stammzellen (iPSC) aus einer seneszenten somatischen Zelle oder aus einer somatischen Zelle von älteren Probanden, das umfasst: Einführen eines Proteins gemäß SEQ ID NO 2 oder eines Nukleinsäuremoleküls, das es kodiert, und eines für EIF3E FL spezifischen Herabregulierers, der die Regulation von Nukleinsäuremolekülen gemäß SE ID NO 1 oder 19, und/oder des Proteins gemäß SEQ ID NO 2 nicht stört, in die Zelle vor und während der Schritte zur zellulären Neuprogrammierung, wobei der EIF3E FL-Herabregulierer eine Nukleinsäure ist, die aus der folgenden Liste ausgewählt wird: eine siRNA, eine shRNA, eine miRNA, ein für EIF3E FL oder Fragmenten davon spezifischer Antikörper, der nicht mit EIF3E-5a reagiert.

13. In-vitro-Verfahren zur Herstellung differenzierter somatischer Zellen, das die folgenden Schritte umfasst:
a) Herstellen humaner oder nicht-humaner induzierter pluripotenter Säugetierstammzellen durch Durchführen des Verfahrens gemäß einem der Ansprüche 7 bis 11 und
b) Unterwerfen der so erhaltenen induzierten pluripotenten Stammzellen eines Protokolls zur Zelldifferenzierung.

14. Verwendung des Proteins gemäß SEQ ID NO 2 oder der Nukleinsäuremoleküle gemäß SEQ ID NO 1 oder 19, oder von anderen Molekülen mit Nukleotidsequenzen, die das Protein gemäß SEQ ID NO 2 kodieren, für die In-vitro-Herstellung von humanen oder nicht-humanen induzierten pluripotenten Säugetierstammzellen.

15. Kit zum Herstellen von humanen oder nicht-humanen induzierten pluripotenten Säugetierstammzellen, das umfasst: eine oder mehrere Aliquote eines Agens gemäß den Ansprüchen 3 bis 7.

## Revendications

1. Molécule d'acide nucléique telle que représentée par la SEQ ID NO : 1 ou 19, codant une isoforme de la protéine EIF3E humaine qui ne comprend pas les exons 6-13.

2. Protéine telle que représentée par la SEQ ID NO : 2, laquelle protéine est une isoforme de la protéine EIF3E humaine (EIF3E-5a).

3. Agent pour la production de cellules CSPI à partir de cellules somatiques de mammifère humain ou non humain comprenant la protéine telle que représentée par la SEQ ID NO : 2 ou une molécule d'acide nucléique telle que représentée par la SEQ ID NO : 1 ou 19, ou une molécule d'acide nucléique codant la protéine de la SEQ ID NO : 2.

4. Agent selon la revendication 3, comprenant en outre un régulateur à la baisse spécifique d'EIF3E de pleine longueur (EIF3E FL) qui n'interfère pas avec la régulation de la SEQ ID NO : 1 ou 19 et/ou de la SEQ ID NO : 2, éventuellement comprenant en outre le facteur de transcription SOX-2 humain, le facteur 4 de type Krueppel humain, le domaine POU, la classe 5, le facteur de transcription 1 et LIN28 ou des acides nucléiques qui les codent, dans lequel ledit régulateur à la baisse d'EIF3E FL est un acide nucléique choisi parmi un ARNsi, un ARNsh, un ARNmi, un anticorps spécifique d'EIF3E FL ou des fragments de celui-ci qui ne réagissent pas avec EIF3E-5a.

5. Agent selon la revendication 4, comprenant en outre la protéine proto-oncogène Myc ou un acide nucléique la codant.

6. Agent selon l'une quelconque des revendications 4 et 5, dans lequel lesdits acides nucléiques sont insérés chacun dans un ou plusieurs vecteurs lentiviraux, ou dans lequel lesdites molécules d'acide nucléique sont des ARNm.

7. Procédé *in vitro* pour la production de cellules souches pluripotentes induites (CSPI) à partir de cellules somatiques, comprenant les étapes suivantes :
a. introduction d'une protéine telle que représentée par la SEQ ID NO : 2 ou d'une molécule d'acide nucléique la codant et, de manière concomitante ou subséquente, le facteur de transcription SOX-2 humain, le facteur 4 de type Krueppel humain, le domaine POU, la classe 5, le facteur de transcription 1 et LIN28 ou des acides nucléiques qui les codent, dans une cellule somatique de mammifère humain ou non humain, et
d. culture de ladite cellule jusqu'à ce qu'elle se divise en une population de cellules pluripotentes,
comprenant éventuellement en outre l'étape b., avant, en même temps que ou après a. et avant d. :
b. introduction de la protéine proto-oncogène Myc ou d'un acide nucléique la codant dans ladite cellule somatique de mammifère humain ou non humain dans un passage de reprogrammation cellulaire,
et/ou comprenant éventuellement en outre l'étape c., avant, en même temps ou après a. et éventuellement c. avant d. :
c. introduction d'un régulateur à la baisse spécifique d'EIF3E FL qui n'interfère pas avec la régulation de la molécule d'acide nucléique telle que représentée par la SEQ ID NO : 1 ou 19, et/ou de la protéine telle que représentée par la SEQ ID NO : 2, dans un passage de reprogrammation cellulaire, lequel régulateur à la baisse d'EIF3E FL est un acide nucléique choisi parmi un ARNsi, un ARNsh, un ARNmi, un anticorps spécifique d'EIF3E FL ou des fragments de celui-ci qui ne réagissent pas avec EIF3E-5a.

8. Procédé selon la revendication 7, dans lequel ladite cellule somatique de mammifère humain ou non humain est une cellule sénescente ou provient d'individus âgés.

9. Procédé selon l'une quelconque des revendications 7 et 8, dans lequel lesdites molécules d'acide nucléique sont respectivement insérées dans un ou plusieurs vecteurs lentiviraux, ou dans lequel lesdites molécules d'acide nucléique sont sous la forme d'ARNm et ladite étape d'introduction dans ladite cellule est réalisée par des transfections transitoires répétées avec lesdites molécules d'acide nucléique.

10. Procédé selon la revendication 8, dans lequel un régulateur à la baisse spécifique d'EIF3E FL qui n'interfère pas avec la régulation de molécules d'acide nucléique telles que représentées par la SEQ ID NO : 1 ou 19 et/ou de la protéine telle que représentée par la SEQ ID NO : 2, comme défini dans la revendication 7, est en outre introduit dans ladite cellule dans un passage de transfection concomitant ou subséquent à l'introduction dudit ARNm codant une protéine telle que représentée par la SEQ ID NO : 2 et avant les étapes de transfection d'ARNm codant le facteur de transcription SOX-2 humain, le facteur 4 de type Krueppel humain, le domaine POU, la classe 5, le facteur de transcription 1, LIN28 et éventuellement la protéine proto-oncogène Myc.

11. Procédé selon la revendication 10, dans lequel ladite cellule somatique de mammifère humain ou non humain est une cellule sénescente ou provient d'individus âgés.

12. Procédé in vitro pour optimiser la production de cellules souches pluripotentes induites (CSPI) à partir d'une cellule somatique sénescente ou à partir d'une cellule somatique provenant d'individus âgés, comprenant l'introduction dans ladite cellule d'une protéine telle que représentée par la SEQ ID NO : 2 ou d'une molécule d'acide nucléique la codant, et d'un régulateur à la baisse spécifique d'EIF3E FL qui n'interfère avec la régulation de molécules d'acide nucléique telles que représentées par la SEQ ID NO : 1 ou 19, et/ou de la protéine telle que représentée par la SEQ ID NO : 2, avant et pendant les étapes de reprogrammation cellulaire, dans lequel ledit régulateur à la baisse d'EIF3E FL est un acide nucléique choisi parmi un ARNsi, un ARNsh, un ARNmi, un anticorps spécifique d'EIF3E FL ou des fragments de celui-ci qui ne réagissent pas avec EIF3E-5a.

13. Procédé *in vitro* pour la production de cellules somatiques différenciées, comprenant les étapes suivantes :
a. production de cellules souches pluripotentes induites de mammifère humain ou non humain par mise en oeuvre du procédé selon l'une quelconque des revendications 7 à 11, et
b. soumission des cellules souches pluripotentes induites ainsi obtenues à un protocole de différenciation cellulaire.

14. Utilisation de la protéine telle que représentée par la SEQ ID NO : 2 ou des molécules d'acide nucléique telles que représentées par la SEQ ID NO : 1 ou 19, ou d'autres molécules avec des séquences de nucléotides codant ladite protéine telle que représentée par la SEQ ID NO : 2 pour la production in vitro de cellules souches pluripotentes induites de mammifère humain ou non humain.

15. Trousse pour préparer des cellules souches pluripotentes induites de mammifère humain ou non humain comprenant une ou plusieurs aliquotes d'un agent tel que défini dans les revendications 3 à 7.


### SEQUENCE LISTING

<110> Centro Nazionale delle Ricerche
<120> NEW METHODS AND AGENTS FOR CELLULAR REPROGRAMMING
<130> BW827R
<160> 19
<170> PatentIn version 3.5
<210> 1
   <211> 812
   <212> DNA
   <213> Homo sapiens
<220>
   <221> CDS
   <222> (29) . . (547)
<400> 1
   gagcacagac tccctttctt ttggcaag atg gcg gag tac gac ttg act act      52
                                  Met Ala Glu Tyr Asp Leu Thr Thr
                                  1               5
   cgc atc gcg cac ttt ttg gat cgg cat cta gtc ttt ccg ctt ctt gaa     100
   Arg Ile Ala His Phe Leu Asp Arg His Leu Val Phe Pro Leu Leu Glu
       10                  15                  20
   ttt ctc tct gta aag gag ata tat aat gaa aag gaa tta tta caa ggt     148
   Phe Leu Ser Val Lys Glu Ile Tyr Asn Glu Lys Glu Leu Leu Gln Gly
   25                  30                  35                  40
   aaa ttg gac ctt ctt agt gat acc aac atg gta gac ttt gct atg gat     196
   Lys Leu Asp Leu Leu Ser Asp Thr Asn Met Val Asp Phe Ala Met Asp
                   45                  50                  55
   gta tac aaa aac ctt tat tct gat gat att cct cat gct ttg aga gag     244
   Val Tyr Lys Asn Leu Tyr Ser Asp Asp Ile Pro His Ala Leu Arg Glu
               60                  65                  70
   aaa aga acc aca gtg gtt gca caa ctg aaa cag ctt cag gca gaa aca     292
   Lys Arg Thr Thr Val Val Ala Gln Leu Lys Gln Leu Gln Ala Glu Thr
           75                  80                  85
   gaa cca att gtg aag atg ttt gaa gat cca gaa act aca agg caa atg     340
   Glu Pro Ile Val Lys Met Phe Glu Asp Pro Glu Thr Thr Arg Gln Met
       90                  95                  100
   cag tca acc agg gat ggt agg atg ctc ttt gac tac ctg gcg gac aag     388
   Gln Ser Thr Arg Asp Gly Arg Met Leu Phe Asp Tyr Leu Ala Asp Lys
   105                 110                 115                 120
   cat ggt ttt agg cag gaa tat tta gat aca ctc tac aga tat gca aaa     436
   His Gly Phe Arg Gln Glu Tyr Leu Asp Thr Leu Tyr Arg Tyr Ala Lys
                   125                 130                 135
   ttc cag tac gaa tgt ggg aat tac tca gga gca gca gaa tat ctt tat     484
   Phe Gln Tyr Glu Cys Gly Asn Tyr Ser Gly Ala Ala Glu Tyr Leu Tyr
               140                 145                 150
   ttt ttt aga gtg ctg atg gag tct tgc tat gtt gcc cag gct gat ctc     532
   Phe Phe Arg Val Leu Met Glu Ser Cys Tyr Val Ala Gln Ala Asp Leu
           155                 160                 165
   aaa ctc ctg gcc tga agtgatcctc cctcctcagc ctttcaatgc tttgggattt     587
   Lys Leu Leu Ala
       170
   acagccatga gcctctgcac cctgcctgtc taagatttct ctagttaatc ctagaagttg   647
   gaaaaagacc ttaaaacaga gactttttgt cttccaacct tggaagagtt tgtttctttt   707
   tgcctttact ttctggtgct tttttcttca cttgtagctc cacatgttgc aaaataaagt   767
   ctcatggctg ctgtcaaaaa aaaaaaaaaa aaaaaaaaaa aaaaa                   812
<210> 2
   <211> 172
   <212> PRT
   <213> Homo sapiens
<400> 2
   Met Ala Glu Tyr Asp Leu Thr Thr Arg Ile Ala His Phe Leu Asp Arg
   1               5                   10                  15
   His Leu Val Phe Pro Leu Leu Glu Phe Leu Ser Val Lys Glu Ile Tyr
               20                  25                  30
   Asn Glu Lys Glu Leu Leu Gln Gly Lys Leu Asp Leu Leu Ser Asp Thr
           35                  40                  45
   Asn Met Val Asp Phe Ala Met Asp Val Tyr Lys Asn Leu Tyr Ser Asp
       50                  55                  60
   Asp Ile Pro His Ala Leu Arg Glu Lys Arg Thr Thr Val Val Ala Gln
   65                  70                  75                  80
   Leu Lys Gln Leu Gln Ala Glu Thr Glu Pro Ile Val Lys Met Phe Glu
                   85                  90                  95
   Asp Pro Glu Thr Thr Arg Gln Met Gln Ser Thr Arg Asp Gly Arg Met
               100                 105                 110
   Leu Phe Asp Tyr Leu Ala Asp Lys His Gly Phe Arg Gln Glu Tyr Leu
           115                 120                 125
   Asp Thr Leu Tyr Arg Tyr Ala Lys Phe Gln Tyr Glu Cys Gly Asn Tyr
       130                 135                 140
   Ser Gly Ala Ala Glu Tyr Leu Tyr Phe Phe Arg Val Leu Met Glu Ser
   145                 150                 155                 160
   Cys Tyr Val Ala Gln Ala Asp Leu Lys Leu Leu Ala
                   165                 170
<210> 3
   <211> 24
   <212> DNA
   <213> Artificial Sequence
<220>
   <223> EIF3E-5a sense primer
<400> 3
   gcatggtttt aggcaggaat attt 24
<210> 4
   <211> 21
   <212> DNA
   <213> Artificial Sequence
<220>
   <223> EIF3E-5a antisense primer
<400> 4
   cttcaggcca ggagtttgag a 21
<210> 5
   <211> 23
   <212> DNA
   <213> Artificial Sequence
<220>
   <223> EIF3E sense (NM_001568)
<400> 5
   cagatgttgg ccatgaatat tga 23
<210> 6
   <211> 20
   <212> DNA
   <213> Artificial Sequence
<220>
   <223> EIF3E antisense (NM_001568)
<400> 6
   gcccagttag gagcctctga 20
<210> 7
   <211> 20
   <212> DNA
   <213> Artificial Sequence
<220>
   <223> GAPDH (NM_002046) sense
<400> 7
   gcaccgtcaa ggctgagaac 20
<210> 8
   <211> 19
   <212> DNA
   <213> Artificial Sequence
<220>
   <223> GAPDH (NM_002046) antisence
<400> 8
   agggatctcg ctcctggaa 19
<210> 9
   <211> 22
   <212> DNA
   <213> Artificial Sequence
<220>
   <223> HPRT1 (NM_000194) sense
<400> 9
   tttgctgacc tgctggatta ca 22
<210> 10
   <211> 28
   <212> DNA
   <213> Artificial Sequence
<220>
   <223> HPRT1 (NM_000194) antisense
<400> 10
   ggtcattaca atagctcttc agtctgat 28
<210> 11
   <211> 18
   <212> DNA
   <213> Artificial Sequence
<220>
   <223> Rpl32 sense (NM_000994)
<400> 11
   ttcatccggc accagtca 18
<210> 12
   <211> 20
   <212> DNA
   <213> Artificial Sequence
<220>
   <223> Rpl32 antisense (NM_000994)
<400> 12
   gtcaatgcct ctgggtttcc 20
<210> 13
   <211> 23
   <212> DNA
   <213> Artificial Sequence
<220>
   <223> Rpl34 sense (NM_000995)
<400> 13
   tttgacatac cgacgtaggc ttt 23
<210> 14
   <211> 20
   <212> DNA
   <213> Artificial Sequence
<220>
   <223> Rpl34 antisense (NM_000995)
<400> 14
   gggttcggga cagcctagtt 20
<210> 15
   <211> 23
   <212> DNA
   <213> Artificial Sequence
<220>
   <223> forward primer for generation of EIF3E-5a RNA (for cell transfection)
<400> 15
   gactcccttt tctttggcaa gat 23
<210> 16
   <211> 24
   <212> DNA
   <213> Artificial Sequence
<220>
   <223> Reverse primer for generation on RNA EIF3E-5a (for cell transfection)
<400> 16
   aggtcttttt ccaacttcta ggat 24
<210> 17
   <211> 19
   <212> RNA
   <213> Artificial Sequence
<220>
   <223> commercial oligo for specific inhibition of EIF3E Full Lenght
<400> 17
   guuuauaugu uaacuuuga 19
<210> 18
   <211> 19
   <212> RNA
   <213> Artificial Sequence
<220>
   <223> Commercial oligo for specific inhibition of EIF3E Full Lenght
<400> 18
   caguguauca gcauuaaca 19
<210> 19
   <211> 812
   <212> RNA
   <213> Homo sapiens
<400> 19
   gagcacagac ucccuuuucu uuggcaagau ggcggaguac gacuugacua cucgcaucgc    60
   gcacuuuuug gaucggcauc uagucuuucc gcuucuugaa uuucucucug uaaaggagau   120
   auauaaugaa aaggaauuau uacaagguaa auuggaccuu cuuagugaua ccaacauggu   180
   agacuuugcu auggauguau acaaaaaccu uuauucugau gauauuccuc augcuuugag   240
   agagaaaaga accacagugg uugcacaacu gaaacagcuu caggcagaaa cagaaccaau   300
   ugugaagaug uuugaagauc cagaaacuac aaggcaaaug cagucaacca gggaugguag   360
   gaugcucuuu gacuaccugg cggacaagca ugguuuuagg caggaauauu uagauacacu   420
   cuacagauau gcaaaauucc aguacgaaug ugggaauuac ucaggagcag cagaauaucu   480
   uuauuuuuuu agagugcuga uggagucuug cuauguugcc caggcugauc ucaaacuccu   540
   ggccugaagu gauccucccu ccucagccuu ucaaugcuuu gggauuuaca gccaugagcc   600
   ucugcacccu gccugucuaa gauuucucua guuaauccua gaaguuggaa aaagaccuua   660
   aaacagagac uuuuugucuu ccaaccuugg aagaguuugu uucuuuuugc cuuuacuuuc   720
   uggugcuuuu uucuucacuu guagcuccac auguugcaaa auaaagucuc auggcugcug   780
   ucaaaaaaaa aaaaaaaaaa aaaaaaaaaa aa                                  812